(19)

Europäisches Patentamt
European Patent Office
Office européen des brevets

(11) **EP 4 437 938 A1**

(12) **EUROPEAN PATENT APPLICATION**

(43) Date of publication:
**02.10.2024 Bulletin 2024/40**

(51) International Patent Classification (IPC):
**A61B 3/028** (2006.01)

(21) Application number: **23305416.2**

(22) Date of filing: **27.03.2023**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Essilor International
94220 Charenton-Le-Pont (FR)**

(72) Inventors:
• **BOUTINON, Stéphane
  75013 PARIS (FR)**
• **MARIN, Gildas
  75012 PARIS (FR)**
• **PELOUX, Marius
  91120 PALAISEAU (FR)**

(74) Representative: **Korakis-Ménager, Sophie
Essilor International
Propriété Intellectuelle
147, rue de Paris
94220 Charenton-le-Pont (FR)**

(54) **DEVICE AND METHOD FOR ESTIMATING A REFRACTIVE ERROR FROM MEASURED VISUAL ACUITY**

(57)     A refractive error of an eye of a user is estimated by receiving measurements (21) of the visual acuity, VA, of the user's eye, called reference VA measurements, and data (22) related to distinct respectively associated levels of blur, called reference blur data, the reference VA measurements being performed within a reference measurement period by imposing the respective levels of blur on the user's eye. A generic model (23) involving a theoretical VA, theoretical blur data and a theoretical refractive error is particularized into a specific model (24) for the user's eye, from the reference VA measurements and the reference blur data, and information (25) relevant to the refractive error of the user's eye is produced from the specific model and provided.

Applications to vision follow-up and to myopia, hyperopia and astigmatism control.

Fig 1

**Description**

**TECHNICAL FIELD**

The invention relates to the domain of vision testing and regards more particularly the estimation of refractive error.

**BACKGROUND ART**

Refractive error pertains to hindered capacity of an eye to focus light accurately on the retina, also referred to as ametropia. It is closely linked to the shape of the eye, notably the cornea and the eyeball length.

Refractive error can be associated with near-sightedness or myopia, corresponding to an excessive eyeball length causing the focusing of light in front of the retina. Distant objects then appear blurry while close objects are clearly seen. Refractive error can alternatively be associated with far-sightedness or hyperopia, corresponding to too short an eyeball length causing the focusing of light behind the retina. Close objects then appear blurry while distant objects are clearly seen. Astigmatism is a type of refractive error in which an eye has a refractive power affected by rotational asymmetry, linked to an improper shape of the cornea, resulting in distorted blurry vision at any distance. As for *presbyopia,* it pertains to deteriorated accommodation ability with aging, prejudicial to clear focusing on close objects.

Being able to diagnose a presence, nature and importance of refractive error constitutes a critical matter for eye care professionals (hereinafter "ECP"), such as orthoptists, optometrists or ophthalmologists. This can rely on objective refraction, exploiting objective measures via dedicated apparatus, e.g. a retinoscope (enabling an examiner to shine light into a patient's eye and observe a reflection off the eye retina) or an autorefractor (a computer-controlled machine measuring how light is affected as it reflects through an eyeball), or on subjective refraction, collecting subjective feedback responses from the patient on the adequacy of lenses having different powers and arranged in front of a tested eye to correct vision. Subjective refraction is typically following objective refraction for refining the obtained measurement results.

The diagnosis of refractive error enables ECPs to decide appropriate management, which may notably include correction via glasses or contact lenses, or refractive surgery. Other provisions may e.g. consist in inciting children to spend more time outdoors for avoiding or slowing worsening of nearsightedness.

While measurements carried out by equipped professionals in a medical setting are indispensable for obtaining relevant and precise diagnoses, autonomous mobile resources adapted to estimate refractive error in other environments and possibly by laymen can be helpful in various circumstances.

For example, intermediary home monitoring of children's eyes by parents between two medical appointments with an ophthalmologist may allow warning in case of significant myopia worsening, and anticipated appointment scheduling if need be.

Also, regularly collected refractive error data, even though coarse approximations, may be helpful to an ECP for enhancing a diagnosis thanks to better grasping of past evolution history.

In addition, in some places or periods where seeing a professional in the medical setting proves difficult for any reasons (e.g. unavailability of professionals, long distances, reduced or impeded mobility), remote online medical consultation based partly on user-estimated refractive errors could be helpful for routine follow-up, front-line checking, or patient orientation.

Several portable solutions have been developed for estimating refractive error. They are most often based in fact on visual acuity or VA measurements, i.e. the ability to recognize small details with precision, expressed as a spatial resolution of a visual processing system. Since ametropia is a common cause of low visual acuity, the refractive error can indeed be usually reflected in the visual acuity, though neural factors may instead or further be involved in the retina (e.g. macular degeneration) or the brain (e.g. damage subsequent to traumatic injury).

VA is typically measured subjectively by relying on optotypes or eye chart symbols, placed at a standardized distance away from a patient for being identified. The smallest reliably recognized symbols are thus considered as representative of the patient's VA. The standardized distance is usually 6 meters in Europe (as stated notably in the European norm EN ISO 8596) and 20 feet in US, for simulating optical infinity.

Refractive error and VA are notably interlinked in that in subjective refraction, the appropriate lens or combination of lenses is the one that provides the best corrected VA or BCVA.

A portable vision testing solution is for example described in patent application WO 2020/176784 to Eyeque Inc. The disclosed apparatus includes a binocular viewer attached to a smartphone, and can implement a combination of front and back lens surfaces, demagnification and other systems to replicate sight lines perceived by a user of a traditional VA test, but in a quite compact way. In addition, an adjustable lens system providing variable optical power can be inserted into the apparatus. A user can thus set the power of the adjustable lens system so as to reach the BCVA, thereby inferring the refractive error (see § 17-18).

Though potentially providing precise refractive error estimations, such a solution requires a dedicated equipment for the user, comprising the specific apparatus and the related adjustable lenses.

In patent application WO 2019/089600 to Welch Allyn Inc., a vision screening apparatus conducts a photorefraction ocular screening test to generate refractive error. The latter is exploited for determining a VA test equivalent, which involves an optotype size adjusted in a Snellen chart testing carried out with the apparatus. That solution can be used for identifying neurological or other issues causing discrepancies between the refractive error measurement and VA examination.

Further to requiring a special equipment, this solution may be costly and difficult to be reliably handled by users.

Patent application WO 2014/164020 to S. P. Lee and A. Dallek proposes computerized refraction and astigmatism determination, not requiring any lenses and possibly performed at home, by relying on proper compliance of a patient with automatic instructions, including distances with respect to a computerized screen (see e.g. § 81-82).

In fact, no effective refractive error estimation is made in this disclosure, which is fully focusing on VA measurements.

Another solution for estimating the refractive error is developed in patent application WO 2014/195951 to O. Limon, O. K. Ancri and A. Zlotnik. The refractive error of an eye of a subject is deduced from a maximum distance of best acuity, or MDBA, which is the maximum distance at which the subject recognizes a displayed sign in a target image. Once the MDBA is properly estimated, the refractive error of the concerned eye is calculated according to the MDBA and to the characteristics of the target image, without requiring the subject to try on different correcting lenses.

That solution offers opportunities to deduce refractive error from VA measurements in a wide range of eye conditions, including myopia, hyperopia, presbyopia and astigmatism. However, the positioning of the subject at the precise required distance, while crucial in the described method, is very sensitive to approximations and errors due to subjective appreciation by the subject (in this respect, see in particular § 49, 107 and 211).

As a matter of fact, though the relationship between refractive error and VA have been scrutinized for a long time, its complexity makes practical transitions from one to the other in user applications quite delicate and prone to errors.

In this respect, it may be referred to the seminal article "Phenomenological model of visual acuity" by J. A. Gómez-Pedrero and J. Alonso, in J. Biomed. Opt., 21(12), 125005, 2016 (hereinafter repeatedly referred to as "the Gómez-Pedrero article"), which describes VA as a function of defocus and pupil diameter, while taking account of astigmatism and BCVA variability among individuals, and to the introduction part of that publication about past related works. Further material may be found e.g. in the article to Essilor International "Influence of combined power error and astigmatism on visual acuity" by C. Fauquier et al., in Vision Science and its Applications, Technical Digest Series (Optica Publishing Group), 1995.

Consequently, estimating refractive error with a portable apparatus in a sufficiently reliable way and without requiring dedicated equipment remains quite challenging. Likewise, safely deducing refractive error from VA measurements is not an easy task.

**SUMMARY**

A purpose of the present disclosure regards estimations of refractive error from visual acuity measurements, in a potentially reliable way not requiring dedicated equipment.

A further purpose of the disclosure is to make possible such estimations by users without medical status or training.

An additional purpose of the disclosure is to enable refractive error estimations by means of a usual mobile device, such as e.g. a smartphone, a tablet or a laptop.

Applications of the disclosure pertaining to refractive error include in particular intermediary monitoring between medical appointments, data history collecting and recording with a view to enhanced medical diagnosis, and remote online medical consultations.

*Preliminary definitions*

The definitions below complete the above.

A "user" is presently defined as a person or an animal having vision capacities, and using the disclosed device or method as a patient or a subject. It may or may not be the person or apparatus operating that device or executing that method.

"Blur" refers to defocus aberration, i.e. an image being out of focus. In the present vision-directed disclosure, blur expresses a subjective perception of refractive error, covering any of the previously mentioned forms. In this context, "blur data" regards data pertaining to type, level and/or source of vision blur.

"Optical power" refers to the degree to which an optical system converges or diverges light. The optical system having a focal length, its optical power is worth the reciprocal of that focal length and in SI units (for "System International") is given in dioptres, i.e. inverse metres. By convention, converging and diverging optical systems have respectively positive and negative optical powers. Consequently, myopia is typically corrected by a negative power lens, while hyperopia or presbyopia is typically corrected by a positive power lens.

The "Minimum Angle of Resolution" or "MAR" is a commonly used scale, which when expressed in arc minutes corresponds to the reciprocal of the VA expressed as a decimal number.

A "visual field" of a user eye is a whole area in which that eye can detect visual stimuli, and a "fixation point" is a particular point at which the user eye is looking in the visual field.

In perimetry (i.e. the measurement of visual functions at topographically defined loci in the visual field), a "meridian" is used for specifying loci in the visual field of a user eye. It corresponds to a polar axis, and thus to a polar angle, in a polar coordinate system having a fixation point as its origin. Consistently, a meridian also amounts to a plane including the corresponding polar axis and an optical axis linking the user eye to the fixation point, or to a plane parallel to the latter.

By convention in this respect, the user having a sagittal plane (i.e. an anatomical plane dividing the user body into right and left sections), a "horizontal" line is defined as a line perpendicular to that sagittal plane. A horizontal meridian is then associated with 0° or 180°, the latter option being adopted in eyewear prescription, and a vertical meridian with 90°.

Refractive error correction in eyewear prescription may be expressed in the form of a "sphero-cylindrical correction". The latter includes a "sphere component" (or "spherical component") which has a same optical power in every direction perpendicular to an optical axis of the eyewear. This can be obtained by a spherical lens, which meets that specificity. In the absence of astigmatism correction, the eyewear correction may be restricted to the sphere component, a related prescription usually including the mention "D.S." (for "Dioptres Sphere") after the sphere power.

In case of astigmatism, the correction further includes an "axis component", given by the meridian associated with the power of the sphere component. That meridian corresponds to the highest correction power or the lowest correction power, depending on the notation, and is expressed in degrees (the polar angle). A "cylinder component" (or "cylindrical component") is given by the offset between the lowest and the highest correction powers. The correction power most remote from the sphere component power is further associated with a +90° meridian shift from the axis component, hereinafter designated as "the perpendicular axis".

More precisely, in the "plus cylinder notation", the cylinder component is positive, so that the axis component and the perpendicular axis give respectively the lowest (most divergent meridian) and highest (most convergent meridian) correction powers. By contrast, in the "minus cylinder notation", the cylinder component is negative, so that the axis component and perpendicular axis give respectively the highest and lowest correction powers.

For an astigmatic user eye, the refractive error correction may also be expressed by a "spherical equivalent", which is a mean power value taking account of both the sphere component and the cylinder component, and may e.g. simply be half-way between the highest and the lowest optical power. It corresponds to mean curvature of a wavefront.

By extension, the refractive error is often designated by the required refractive error correction, which convention will be adopted below.

As mentioned above, an optotype is an eye chart symbol placed at a standardized distance away from a patient for being identified. In the following, this term will more generally be used for designating a representation shown to the patient in a chart at a given effective or simulated distance (e.g. by virtual reality) for identification or perception, possibly including one or more symbol and/or grating, having given shape, size and orientation.

In what follows, an "oriented test target" is an optotype or a set of optotypes displayed on an eye chart and differentiated with respect to at least one particular direction corresponding respectively to at least one particular meridian (i.e. polar angle), proper to determining an axis component and/or a cylinder component of a refractive error for an eye of an astigmatic user. The oriented test target may e.g. include parallel lines adapted to be rotated around an optical axis, or lines evenly spaced at regular angle intervals. By contrast, a "non-oriented test target" designates an optotype or a set of optotypes displayed on an eye chart and not specifically differentiated with respect to particular directions with a view to determining an axis component and/or a cylinder component. Such a non-oriented test target may be relevant to determining a sphere power of a non-astigmatic user, or a sphere equivalent of an astigmatic user.

"Accommodation" is a process by which a user eye changes optical power to maintain a clear image or focus of an object as its distance varies. The capability of a subject to accommodate, namely the accommodation amplitude, is quantified in dioptres by the difference between the optical power for closest clear vision, i.e. for the "punctum proximum or PP", and the optical power for farthest clear vision, i.e. for the "punctum remotum or PR", the PR and PP delimiting the accommodation area.

A "pupil" is a hole of a user eye through which light can strike the retina, surrounded by an "iris" being a contractile structure regulating the size of the pupil and hence the amount of light received by the retina. The pupil thus functions like a diaphragm aperture of an optical system limiting the size of a light beam entering the eye.

The terms "adapted" and "configured" are used in the present disclosure as broadly encompassing initial configuration, later adaptation or complementation of the present device, or any combination thereof alike, whether effected through material or software means (including firmware).

The term "processor" should not be construed to hardware capable of executing software, and refers in a general way to a processing device, which can for example include a computer, a microprocessor, an integrated circuit, or a programmable logic device (PLD). Additionally, the instructions and/or data enabling execution of associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM memory. Instructions may be notably stored in hardware, software, firmware or in any combination thereof.

### Objects of the disclosure

The disclosure concerns a device for estimating a refractive error of an eye of a user from visual acuity of the user's eye. The device includes:

- at least one input adapted to receive at least two measurements of the visual acuity of the user's eye, called reference VA measurements, and data related to at least two distinct levels of blur respectively associated with the reference VA measurements, called reference blur data, the reference VA measurements being performed within a reference measurement period by imposing the respective levels of blur on the user's eye,
- at least one processor configured for particularizing for the user's eye into a specific model, from the reference VA measurements and the reference blur data, a generic model involving theoretical visual acuity, theoretical blur data and a theoretical refractive error, and for producing information relevant to the refractive error of the user's eye from that specific model,
- at least one output adapted to provide the information relevant to the refractive error of the user's eye.

The reference blur data are thus blur data having the particularity to be related to two or more distinct levels of blur, each associated with a VA measurement (or more) of the user's eye.

The reference measurement period is advantageously chosen short enough to avoid physiological evolution of the refractive error of the user's eye during that period, and in some embodiments, as short as possible so as to favour homogeneous conditions over the VA measurements. In particular modes, it is thus smaller than one day, advantageously smaller than one hour, and still more advantageously, smaller than 10 minutes.

It may on the other hand be chosen long enough to facilitate the user's testing efforts by spreading them over time and/or to enable repeated similar testing for proper averaging. Such averaging may be useful to reflect more accurately the effective current refractive error of the user's eye, and to avoid e.g. result distortion due to user fatigue, user distraction or disturbed environment. The averaged VA measurements may be carried out in similar environment, ambient lighting, day hour and/or user physical condition. It may instead be purposely carried out in different conditions for same or similar levels of blur, so as to mitigate the influence of related factors.

Accordingly, in particular modes, the reference measurement period is comprised between one day and one week.

The specific model is an expression of one or more relationships between the visual acuity and the refractive error of the user's eye, compliant with the generic model having one or more parameters, in which at least some of those parameters are set. A parameter is defined as a variable or multi-value element relevant to the generic model, which can be determined or evaluated. It may notably be a term in a function representing the generic model, the value of which singles out a specific form of the function among two or more forms, or an independent variable in a set of parametric equations. It may also be an indicator determining a content of the generic model among two or more possible contents. For sake of clarity, it is observed that the concerned parameters do not include the measured visual acuity nor the desired refractive error. The reference blur data may include parameter values directly relevant to one or more of the generic model parameters. The specific model is derived from the generic model by reducing the number or extension field of one or more parameters.

In some implementations, the produced information relevant to the refractive error of the user's eye corresponds to an estimation of the refractive error during the reference measurement period. Accordingly, unknowns in the generic model (including parameters and refractive error) are expressly or implicitly obtained or turned down, whether notably by direct entry, determination, estimation, elimination (e.g. in a system of equations), realistic guess, or random selection in a given range. In other implementations, that produced information corresponds to one or more parameter values of the specific model, which can be subsequently exploited for estimating the refractive error from visual acuity measurements after the reference measurement period.

Thanks to the exploitation of the generic model, and based on those reference blur data together with the associated VA measurements, estimations of the refractive error and not only hints to the refractive error can be produced.

In addition, by contrast with the solution developed in WO 2014/195951 in which the calculation of the refractive error is deduced from the MDBA, the estimation of the refractive error using the device of the present disclosure notably relies on at least two VA measurements respectively associated with distinct levels of blur.

The disclosed device may, in some implementations, provide accurate and precise refractive error estimations, even possibly where subject to limited available precision levels in individual VA measurements, thanks to the consideration of two or more blur levels.

As the skilled person will note, that solution unexpectedly moves away from the known processes and theoretical approaches proposed so far, which in closest configurations establish a one-to-one correspondence between VA and refractive error subject to specific conditions - such as e.g. best acuity, or given distance and pupil size.

Also, the device of the disclosure may be applicable to follow-up of various vision conditions of the user, including myopia, hyperopia, astigmatism or presbyopia.

By enabling the estimation of refractive error through VA measurements, the device of the disclosure may, in some modes, allow users to proceed with refractive error estimation outside a medical setting, including possibly at home, and/or may be relevant to correct operation by users without medical status or training. Such VA measurements may be performed e.g. by means of portable or handheld devices such as smartphones, tablets or laptops.

The visual acuity of a person may for example be evaluated as described in patent application WO 2022/013410 to Essilor International. According to that disclosure, the user is positioned in front of a mirror at a predefined distance, a mobile device including a camera and a screen is arranged with its front camera facing the mirror, a distance between that front camera and a virtual image of the mobile device in the mirror is measured, optotypes are displayed on the screen of the mobile device, and the user VA is evaluated when viewing the optotypes.

As suggested above, the device of the disclosure may therefore be relevant to intermediary monitoring between medical appointments, data history collecting and recording with a view to enhanced medical diagnosis, or remote online medical consultations. It may also prompt a user to schedule a new appointment to an ECP when significant VA changes are identified as needing a new refractive error correction. In myopia cases in children, it may further enable myopia control actions to be triggered. The latter may include regularly spending more time outdoors and/or wearing dedicated glasses directed to slowing down myopia progression, like those incorporating the lenses commercialized by the present Applicant under the name "Stellest™" (which rely on highly aspherical contiguous lenslet distribution). The device makes also possible efficiency follow up of such actions.

In some implementations, the processor(s) is/are configured for determining further items of the reference blur data, which can be exploited for obtaining further associated items of the reference VA measurements, those further items of the reference blur data and of the reference VA measurements being then input to the processor(s) for producing refined and/or completed items of the information relevant to the refractive error. Such a feedback mechanism may be iterated one or more times, as appropriate in view of the used generic model and/or of a target level of accuracy or precision.

In a first (above-mentioned) category of modes, the produced information relevant to the refractive error includes an estimation of the refractive error of the user's eye during the reference measurement period.

In a second (above-mentioned) category of modes, which can be combined in a same device with modes of the first category, the produced information relevant to the refractive error includes at least one parameter value relevant to the specific model. That or those parameter value(s) enable an estimation of the refractive error of the user's eye after the reference measurement period, called later refractive error estimation, to be produced from at least one measurement of the visual acuity of the user's eye after the reference measurement period, called later VA measurement.

In this respect, the specific model may be entirely or partly established during the reference measurement period, and exploited later on for estimating refractive error evolutions over time.

The parameter value(s) may further be determined again at a later time after the reference measurement period, in updating the specific model so as to take account of physiological evolution. Such updating may be repeated whenever deemed useful or convenient, e.g. at regular time intervals or during ECP consultations.

In particular implementations of the second category of modes, the input(s) is/are adapted to receive the later VA measurement(s), the processor(s) is/are configured for producing the later refractive error estimation from the specific model and from the later VA measurement(s), and the output(s) is/are adapted to provide the later refractive error estimation.

Namely, the device is then configured for executing itself the production of later refractive error estimations.

In variants, the device is adapted to make the parameter value(s) relevant to the specific model available to an external module in charge of producing the later refractive error estimation (directly or e.g. via a storage unit). In some modes, the device may then not be configured itself for such estimations. For example, two distinct components are used, one for determining the specific model during the reference measurement period, and another for exploiting that specific model during a later period.

In some implementations of the second category of modes, the reference blur data include at least one measurement of the refractive error of the user's eye during the reference measurement period, called reference refractive error measurement.

Such measurement of the refractive error is thus entered into the device for being exploited in particularizing the generic model so as to obtain later estimations of the refractive error after the reference measurement period. In more particular implementations, the estimation of the refractive error during the reference measurement period is looped back to the reference refractive error measurement, so as to adjust or refine the specific model or to proceed with model calibration, which may e.g. be done iteratively.

The device may produce the later refractive error estimation by computing a refractive error deviation with respect to the reference refractive error measurement in function of a deviation of the later VA measurement with respect to the reference VA measurement.

In some other modes, the levels of blur are imposed at least partly by performing the reference VA measurements at respectively two or more reference distances from the user's eye to a test target, and the theoretical blur data include a theoretical distance from the user's eye.

In more particular implementations, the generic model involves successively, starting and moving away from the user's eye, three areas relevant to the theoretical VA of the user's eye and consisting in a closest area, an accommodation area and a farthest area. The accommodation area is associated with a flat range of the theoretical VA, and the closest area and farthest area are associated with variable values of the theoretical VA. The levels of blur are then imposed at least partly by performing the reference VA measurements at respectively those reference distances including at least one distance corresponding to the accommodation area and at least one distance corresponding to the closest area and/or the farthest area.

By convention, the farthest area may be possibly carried forward to infinity or even be virtual (i.e. optically behind the user's eye), which means that only the closest area and the accommodation area are then effectively accessible without added lenses. This can reflect cases where the user's eye is emmetropic or hyperopic, or where it is presbyopic without myopia.

For example, the device being particularly suited to myopia follow-up, the reference VA measurements are performed at one distance in the accommodation area and two distances in the farthest area. In another example in which the device is particularly suited to presbyopia or hyperopia follow-up, the reference VA measurements are performed at one distance in the accommodation area and two distances in the closest area. In additional examples focused on hyperopia, the reference VA measurements are performed at at least one distance in the accommodation area while imposing a positive optical power correction on the user's eye, besides possibly also at at least one distance in the closest area - as developed below.

In some further modes, which may be combined with the previous ones, at least one of the levels of blur is imposed at least partly by performing at least one of the reference VA measurements at respectively at least one reference direction angle of an oriented test target, the theoretical blur data include a theoretical direction angle with respect to a predetermined axis, and the processor(s) is/are configured for particularizing the generic model also from the reference direction angle(s).

The device is then configured for matching the one or more reference direction angle(s) with the theoretical direction angle in particularizing the generic model into the specific model.

For example, the reference VA measurements are performed at two distinct reference direction angles of an oriented test target, those direction angles being possibly orthogonal, i.e. ± 90° apart.

In particular implementations of the modes involving the oriented test target, the refractive error of the user's eye having a spherical component, a cylindrical component and a cylinder axis, the theoretical refractive error has a theoretical spherical component, a theoretical cylindrical component and a theoretical cylinder axis. The device is then such that:

- the input(s) is/are adapted to receive at least one of the reference VA measurements with respect to a non-oriented test target, the reference VA measurement(s) at respectively the reference direction angle(s) of the oriented test target and the respectively associated reference blur data, the reference blur data further including information on at least one offset of the cylinder axis with respect to the predetermined axis,
- the processor(s) is/are configured for particularizing the generic model into the specific model from those reference VA measurements with respect to both the non-oriented test target and the oriented test target and from the reference blur data, and for producing information relevant to the spherical component and the cylindrical component of the refractive error of the user's eye,
- the output(s) is/are adapted to provide the information relevant to that spherical component and that cylindrical component.

Thereby combining the exploitation of the non-oriented test target and of the oriented test target may prove quite efficient in some implementations. Notably, the non-oriented test target may be used for deriving a spherical equivalent of the refractive error of the user's eye, while the oriented test target may be used for deriving the cylindrical component, thereby for deducing the spherical component - which may e.g. be given by the difference between the spherical equivalent and half the cylindrical component.

In particular modes, at least one of the levels of blur is imposed at least partly by performing at least one of the reference VA measurements with respectively at least one lens arranged in front of the user's eye so as to cause at least one predetermined optical power correction, and the theoretical blur data include a theoretical optical power correction imposed on the user's eye.

The lens(es) may have a positive optical power, in which case the images of the PP and PR through the lens(es) are moved closer to the user's eye, or have a negative optical power, in which case the images of the PP and PR through the lens(es) are moved farther from the user's eye.

In more specific embodiments, in the generic model, a variation of the theoretical refractive error is equated to the theoretical optical power correction.

This amounts to anticipating potential evolutions of the refractive error by equating a relationship linking a refractive error deviation over time to a VA deviation over time on one hand, with a relationship linking an imposed refractive error offset to a thereby induced VA variation during the reference measurement period on the other hand.

In some modes:

- the input(s) is/are adapted to receive dimensional data on a pupil of the user's eye during the reference VA measurements, called reference pupil data,
- the generic model involving a theoretical pupil dimension, the processor(s) is/are configured for particularizing the generic model into the specific model also in function of the reference pupil data.

The pupil dimensional data may in other modes, possibly combined with the previous ones, be exploited as at least part of the reference blur data, i.e. include distinct values respectively associated with two or more reference VA measurements and relied on for particularizing the specific model.

In addition, the disclosure concerns an automatic module for myopia control, including a device for estimating a refractive error of an eye of a user according to any of the modes of the disclosure.

The disclosure also pertains to an apparatus chosen among a smartphone, a tablet, a laptop and a virtual reality head-mounted display (HMD) system, comprising a device for estimating a refractive error according to any of the modes of the disclosure.

The disclosure further relates to a method for estimating by at least one processor a refractive error of an eye of a user from visual acuity of the user's eye. According to the disclosure, the method includes:

- receiving at least two measurements of the visual acuity of said user's eye, called reference VA measurements, and data related to at least two distinct levels of blur respectively associated with the reference VA measurements, called reference blur data, the reference VA measurements being performed within a reference measurement period by imposing the respective levels of blur on the user's eye,
- particularizing for the user's eye into a specific model, from the reference VA measurements and the reference blur data, a generic model involving a theoretical visual acuity, theoretical blur data and a theoretical refractive error, and producing information relevant to the refractive error of the user's eye from that specific model,
- providing the information relevant to the refractive error of the user's eye.

The method for estimating a refractive error is advantageously executed by a device for estimating a refractive error compliant with any of the modes of the disclosure.

In addition, the disclosure relates to a computer program comprising software code adapted to perform a method for estimating a refractive error compliant with any of the above execution modes when the software code is executed by a processor.

The present disclosure further pertains to a non-transitory program storage device, readable by a computer, tangibly embodying a program of instructions executable by the computer to perform a method for estimating a refractive error, compliant with the present disclosure.

Such a non-transitory program storage device can be, without limitation, an electronic, magnetic, optical, electromagnetic, infrared, or semiconductor device, or any suitable combination of the foregoing. It is to be appreciated that the following, while providing more specific examples, is merely an illustrative and not exhaustive listing as readily appreciated by one of ordinary skill in the art: a portable computer diskette, a hard disk, a ROM (read-only memory), an EPROM (Erasable Programmable ROM) or a Flash memory, a portable CD-ROM (Compact-Disc ROM).

## LIST OF FIGURES

The present disclosure will be better understood, and other specific features and advantages will emerge upon reading the following description of particular and non-restrictive illustrative embodiments, the description making reference to the annexed drawings wherein:

**Figure 1** is a block diagram schematically representing a particular mode of a device for estimating a refractive error of an eye of a user, compliant with the present disclosure;

**Figure 2** shows the device of Figure 1 in a first category of implementations, in which an estimation of the refractive error can be directly obtained during a reference measurement period;

**Figure 3** shows the device of Figure 1 in a second category of implementations, in which a specific model can be obtained during a reference measurement period, together with a block diagram of a further device configured for exploiting the specific model at a later time so as to obtain an estimation of a later refractive error;

**Figure 4** is a block diagram schematically representing a device corresponding to the second category of implementations of Figure 3 while also encompassing the functionalities of the further device;

**Figure 5** illustrates the exploitation over time of the device of Figure 3 or of Figure 4, based on the reference measurement period and on multiple later measurement periods;

**Figure 6** shows an example curve of a VA variation of an eye of a myopic user in function of a distance to the user's eye, and indicates possible positions selected on the curve in exploiting a particular embodiment of the device of Figure 1;

**Figure 7** shows typical curves of MAR variations of an eye of a myopic user in function of the reciprocal of a distance to the user's eye, depending on a parameter reflecting a tolerance to defocus in a generic model potentially exploited with the device of Figure 1;

**Figure 8** shows an example curve of a VA variation of an eye of a hyperopic user in function of the reciprocal of a distance to the user's eye, and indicates possible positions selected on the curve in exploiting a particular embodiment of the device of Figure 1;

**Figure 9** shows example curves of VA variations of an eye of an astigmatic and myopic user in function of a distance to the user's eye, corresponding respectively to mean VA, VA along the astigmatism axis, and VA along an axis perpendicular to the astigmatism axis, as possibly exploited in particular embodiments of the device of Figure 1;

**Figures 10A, 10B, 10C, 10D, 10E** and **10F** represent example optotypes used in measuring astigmatism with an implementation of the device of Figure 1, and directed to estimating a mean VA value corresponding to a spherical equivalent of refractive error (with Figure 10A), an astigmatism axis, and axial VA values;

**Figure 11** shows a typical curve of MAR variation of an eye of a user in function of a diameter of the pupil of that user's eye, as possibly exploited in particular embodiments of the device of Figure 1;

**Figures 12A and 12B** represent two respective steps carried out with a handheld apparatus and a mirror for monitoring a pupil diameter of an eye of a user when exploiting the device of Figure 1;

**Figure 13** is a flow chart showing method steps executed with the device of figure 1;

**Figure 14** diagrammatically shows an apparatus incorporating the device represented on figure 1.

On the figures, identical or similar elements are designated by the same references.

## ILLUSTRATIVE EMBODIMENTS

The present description illustrates the principles of the present disclosure. It will thus be appreciated that those skilled in the art will be able to devise various arrangements that, although not explicitly described or shown herein, embody the principles of the disclosure and are included within its spirit and scope.
All examples and conditional language recited herein are intended for educational purposes to aid the reader in understanding the principles of the disclosure and the concepts contributed by the inventor to furthering the art, and are to be construed as being without limitation to such specifically recited examples and conditions.
Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

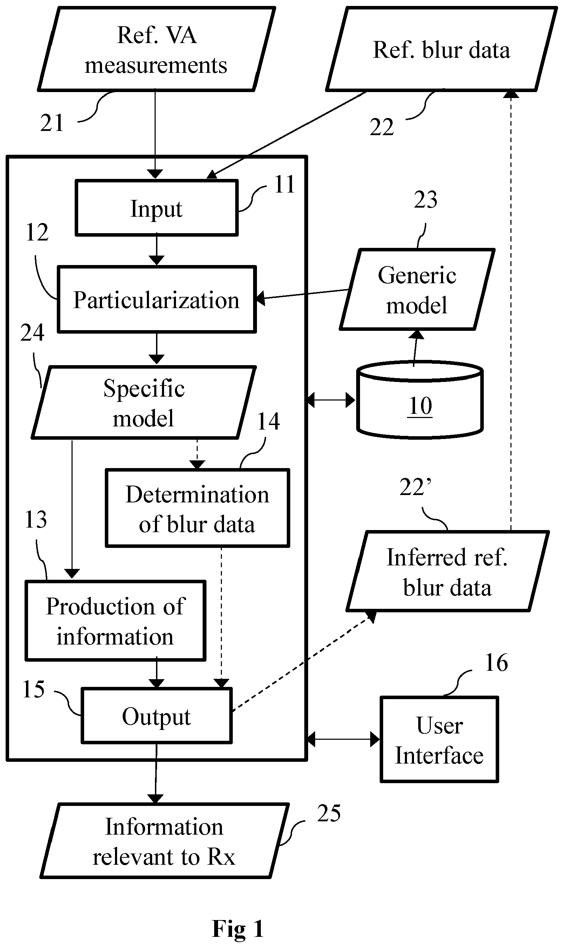

The present disclosure will be described in reference to a particular functional embodiment of a device 1 for estimating a refractive error of an eye of a user from VA of that eye, as illustrated on **Figure 1.**

Though the device 1 is described with reference to a particular eye of a user, it may be adapted to each of two eyes of the user and/or to multiple users.

The refractive error of the user's eye, noted "Rx" for brevity, may be given by one or more values. For example, an eye subject to myopia or hyperopia without astigmatism may have its refractive error represented by a unique value typically expressed in dioptres, which corresponds to spherical power error (positive defocus for myopia, negative defocus for hyperopia, and conversely for needed corrections). In the further presence of regular astigmatism, three values could be appropriate, giving respectively sphere, cylinder and axis components (typically expressed in dioptres for the sphere and cylinder components, and in degrees for the axis component). In the presence of higher-order aberrations, additional values may be required, e.g. coefficients of Zernike polynomials - as e.g. described in the article "Eye aberrations analysis with Zernike polynomials" by V. V. Molebny et al., Proceedings of SPIE, 3246, June 1998.

The device 1 is advantageously an apparatus, or a physical part of an apparatus, designed, configured and/or adapted for performing the mentioned functions and produce the mentioned effects or results. In alternative implementations, the device 1 is embodied as a set of apparatus or physical parts of apparatus, whether grouped in a same machine or in different, possibly remote, machines.

The device 1 is adapted to receive measurements 21 of the visual acuity of the user's eye, called reference VA measurements, during a reference measurement period T0.

In advantageous execution modes, the period T0 is smaller than 10 minutes, so that the user and environmental conditions can remain stable during the measurements. However, the period T0 may be extended in some implementations, for example between 10 minutes and 30 minutes, or between 30 minutes and 1 hour, so as to leave time between monitoring operations for checking and adjusting some measurement aspects before resuming operations. Such extended time may possibly be useful in complex measurements (e.g. in case of astigmatism), so as to avoid too demanding efforts by the user during a short timeframe. Alternatively, or additionally, it may be exploited for averaging results for sake of enhanced precision, e.g. for mitigating disturbances due to uncontrolled user-related or external factors. In some embodiments, the period T0 is extended between 1 hour and 1 day, or even between 1 day and 1 week. In the latter case, it may be appropriate to proceed with similar measurements at approximately a same hour during two or more consecutive days, so as to reach close respective measurement conditions, and to proceed with proper averaging of obtained results over those days. At any rate, the period T0 is advantageously chosen short enough to avoid physiological evolution of the refractive error of the user's eye during that period, which may depend on specificities of the user's eye.

In addition to the reference VA measurements 21, the device 1 is adapted to receive during that period T0, data 22 related to at least two distinct levels of blur respectively associated with the VA measurements 21, and imposed on the user's eye for obtaining the respectively associated VA measurements 21. Those data 22, called reference blur data, may take various forms, including notably distances to the user's eye, direction angles of oriented test targets, dimensional pupil data, refractive power of added lenses, and measured refractive errors, as will be developed below.

The reference VA measurements 21 and reference blur data 22 may be user-entered, streamed, transmitted to the device 1 via a telecommunication network, such as e.g. a wireless local network, a cell network or a wired network, or retrieved from a database.

The device 1 is further adapted to get a generic model 23, which may notably be stored in one or more local or remote database(s) 10, and retrieved from it or them as and when appropriate. The database(s) 10 can take the form of storage resources available from any kind of appropriate storage means, which can be notably a RAM or an EEPROM (Electrically-Erasable Programmable Read-Only Memory) such as a Flash memory, possibly within an SSD (Solid-State Disk).

That generic model 23 involves theoretical VA, theoretical blur data and a theoretical refractive error. It is provided for serving as a basis in generating information 25 relevant to the refractive error of the user's eye, by mapping the entered reference VA measurements 21 and the reference blur data 22 to respectively the theoretical VA and the theoretical blur data, and by inferring one or more estimations of the refractive error of the user's eye from the theoretical refractive error. Accordingly, the reference VA measurements 21 and the reference blur data 22 are in line with the used generic model 23 and are correspondingly prepared. Also, though the use of one generic model is developed in the present disclosure, two or more generic models may be available to the device 1 and exploited successively or in parallel.

The generic model 23 may be implicitly available to the device 1 instead of being expressly obtained, via proper provided parameter inputs and related operations. Such operations may e.g. include any type, number and combination of additions or subtractions, multiplications or divisions, exponentiations with integer exponents or rational exponents (i.e. with roots), trigonometric transformations, logarithms, extractions from look-up tables, deductions by artificial intelligence - whether via expert systems or machine learning - and others. In some particular modes, the operations may for instance consist in a single multiplication or division based on a single parameter, while in others, they may involve a complex combination of rational, circular and exponentiation functions, applied to more than 3, more than 5, or even more than 7 unknowns (including parameters and components of the theoretical Rx components but not counting the theoretical VA).

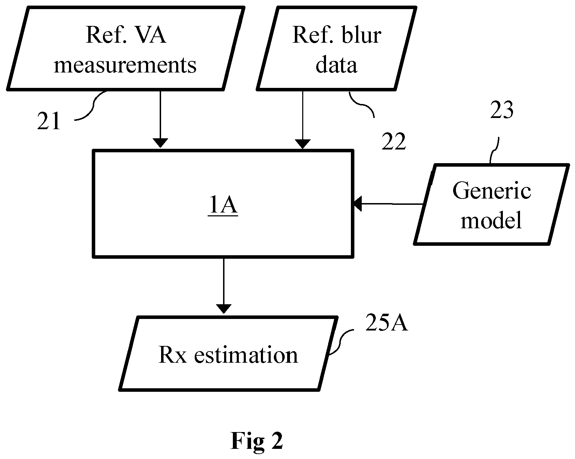

In some implementations, as illustrated on **Figure 2** with the device 1 noted 1A, the information 25 relevant to the refractive error of the user's eye corresponds to pieces of information 25A pertaining to that refractive error during the reference measurement period T0. In particular, the information 25 may give one or more estimations of that refractive error during the period T0.

In other implementations, the information 25 includes parameter values enabling the generic model 23 to be specified or refined so as to make it more relevant to the user's eye, with a view to Rx estimations during the period T0 by another entity than the device 1 or to future Rx estimations after the end of the period T0. Those values relate to all of part of parameters of the generic model 23, and once completely specified, the model may notably be used by the device 1 or another device to infer future Rx estimations from later visual acuity measurements following the period T0.

Such a two-step process may facilitate a follow-up of physiological properties of the user's eye overtime, based on possibly precise initial monitoring. Namely, the future Rx estimations may be performed at various points of time after the period T0. In addition, the generic model 23 may be specified or refined again after the period T0, so as to take account of evolutions of the user's eye capacities. This could be performed any number of times, and possibly lead to regular model updating corresponding to successive reference measurement periods, between which any number of Rx estimations can be carried out on the ground of the most recent model updating.

For example, the reference measurement period T0 is smaller than one hour, and the subsequent Rx estimations relying on the parameter values established during the period T0 are effected every week, fortnight or month, depending on the kind of followed-up user's eye condition, e.g. myopia, presbyopia or astigmatism, and its evolution speed. The frequency of the subsequent Rx estimations may be adapted to the previously monitored evolutions, being higher when the evolution is steeper and lower when the evolution is slower. The reference measurements may e.g. be renewed every month while the subsequent Rx estimations are performed every week.

In still other implementations, the device 1 combines the previous capacities, and is thus adapted to provide information pertaining to the refractive error of the user's eye during the reference measurement period T0 and to specify or refine the generic model 23 for future estimations after the period T0 as well.

In some modes (**Figure 1**), the device 1 is configured for outputting not only the information 25, but also inferred reference blur data 22'. The latter can then be exploited themselves within the reference blur data 22, by measuring visual acuity of the user's eye corresponding to those inferred reference blur data 22' and hence obtaining further items of the reference blur data 22. A feedback loop is thus constituted, in which those operations may be effected just once or repeated any number of times. A progressive interactive refinement of the obtained results during the period T0 and involving successive VA measurements is thus feasible, in a possibly iterative way.

In such a process, the reference blur data 22 and associated reference VA measurements 21 may be initially set with a limited number of items, e.g. corresponding to a single level of blur, and be completed or progressively grown with the inferred reference blur data 22' and the associated VA measurements. Alternately, the reference blur data 22 and associated reference VA measurements 21 may be initially set as a whole and progressively adjusted based on the inferred reference blur data 22'.

In particular implementations, the device 1 is configured for proceeding with averaging of two or more values of at least one same item of the information 25 produced during the period T0, which item may be an estimation of the refractive error. Such averaging enables potential variances, fluctuations and errors linked to the user and/or the environment to be taken into account, with a view to obtaining more reliable values. Successive VA measurements are thus performed on the ground of the same reference blur data 22 (or of the same initial reference blur data 22 if a feedback loop is exploited), leading to respective items of the information 25. For example, those measurements and information production are executed consecutively within less than 30 minutes, and/or on consecutive days at approximately a same hour if the period T0 covers several days. The averaging may without limitation consist in an arithmetic mean, a geometric mean, a harmonic mean, a median, or a quadratic mean (i.e. root mean square). Also, the values to be averaged may be weighted, e.g. for giving more weight to a first measurement compared with following ones having a corrective function.

The device 1 is interacting with a user interface 16, via which information can be entered and retrieved by a user. The user interface 16 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system. The interactions with the user interface 16 may be indirect, for example where the device 1 takes the form of a hardware component, such as whole or part of an integrated circuit embedded in an apparatus.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. The modules belonging to a device are preferably embodied within at least one processor of that device.

The device 1 includes an input module 11 adapted to receive the reference VA measurements 21 and the reference blur data 22. As described above, those may be possibly derived from the inferred reference blur data 22'. The device 1 also includes an output module 15 adapted to provide the information 25 and, if relevant depending on the implementations, the inferred reference blur data 22'.

In addition, a particularization module 12 of the device 1 is tasked with particularizing the generic model 23 for the user's eye into a specific model 24 adapted to be used for producing the information 25 (and possibly the inferred reference blur data 22'). The specific model 24 can be considered as an outcome of specifying or refining the generic model 23 as fed by the reference VA measurements 21 and the reference blur data 22.

In some implementations, the specific model 24 is completely set by the particularization module 12 during the period T0, possibly following one or more iterations involving the inferred reference blur data 22' and/or an averaging step. The completely set model 24 may then be used for producing estimations of the refractive error of the user's eye during the period T0 and/or after this period T0. The specific model 24 is completely set insofar as its parameters have known values, which allow estimations of the refractive error from VA measurements associated with blur data.

Though fully available for estimations, the complete specific model 24 may be updated over time, for taking account of physiological evolutions of the user's eye.

Also, the specific model 24 may be purposely loosened so as to enable adjustment of one or more parameters, thereby used as variables. For example, the generic model depends on a distance to the user's eye, a direction angle with respect to an oriented test and/or a pupil diameter. While the latter have specified values in the complete specific model 24, at least part of them are left flexible so as to reflect effective later measurement conditions.

In addition, partial achievement of the specific model 24 may be completed during the reference period T0 by another entity than the device 1.

Downstream of the particularization module 12, an information production module 13 is in charge of producing the information 25, and where provided for, a blur data determination module 14 is in charge of producing the inferred reference blur data 22'. Though presented separately as distinct functional entities, the modules 12, 13 and 14 may in fact relate to a same set of operations applied to the reference VA measurements 21 and the reference blur data 22 to produce the information 25 - and possibly the inferred reference blur data 22'.

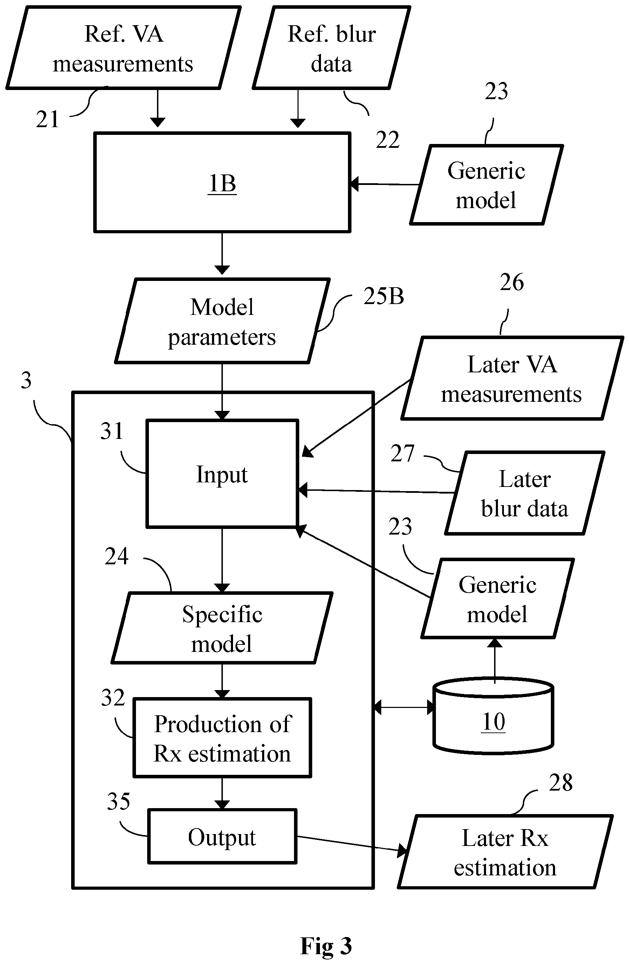

In implementations in which the information 25 includes parameter values 25B, as illustrated on **Figure 3** with the device 1 noted 1B, another device 3 is in charge of inferring a later Rx estimation 28 from later visual acuity measurements 26 and associated later blur data 27, based on the generic model 23 and those parameter values 25B. In particular embodiments, the device 1B is also configured for providing the Rx estimation 25A during the period T0, further to providing the parameter values 25B.

The parameter values 25B may be obtained or retrieved by the device 3 in any way, such as notably by extracting them from a storage source, e.g. the database(s) 10, where they were previously recorded from the device 1, or by receiving them from the device 1 on demand.

The later blur data 27 are adapted to the specific model 24, and enable straightforward inference of Rx estimations from the associated later VA measurements 26.

The device 3 includes an input module 31 for receiving the later VA measurements 26 and the later blur data 27 and an output module 35 for providing the later estimation 28 of the refractive error of the user's eye. It further incorporates a module 32 in charge of producing the later Rx estimation 28 from the later VA measurements 26 and the later blur data 27, based on the specific model 24 induced by the parameter values 25B and the generic model 23. The device 1B may be particularly interesting for getting an accurate version of the specific model 24, for example based on professional equipment in medical premises under the responsibility of an ECP and/or substantial computation capacities of a workstation, the device 3 being possibly a mere mobile apparatus enabling a layman to obtain relatively high-level precision estimations of the refractive error from simple operations hinging on the available specific model 24.

In alternative embodiments, in which the specific model 24 is completely set, the later blur data 27 are not provided to the device 3, but the VA measurement conditions are compliant with the reference blur data 22 (e.g. distance, direction angle, pupil diameter) associated with that model 24.

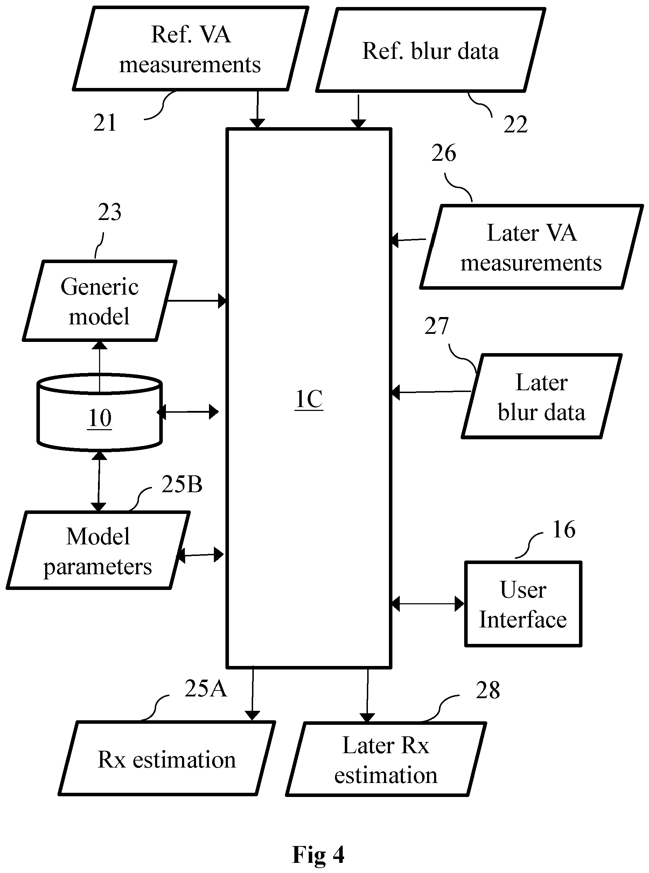

In other implementations of the device 1, noted 1C and illustrated on **Figure 4,** in which the information 25 includes parameter values 25B determining the specific model 24 during the period T0, the device 1C is adapted to proceed itself with generating the parameter values 25B determining the specific model 24 during the period T0 and with subsequently exploiting those parameters values 25B for producing the later Rx estimation 28 from the later visual acuity measurements 26 and associated later blur data 27.

The device 1C may e.g. be implemented in a mobile apparatus potentially offering stand-alone capacities of prompt relatively precise Rx estimations from simple VA measurements, based on periodic more deepened measurements.

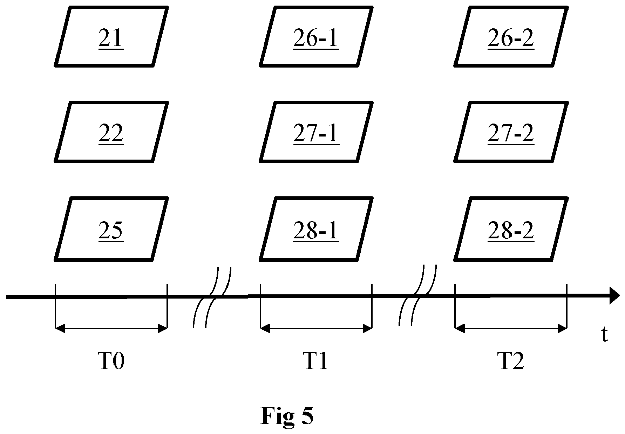

In operation, whether with the combination of the devices 1B and 3 or with the device 1C, as visible on **Figure 5,** the parameter values 25B and possibly the Rx estimation 25A are produced during the reference measurement period T0 from the reference VA measurements 21 and the reference blur data 22. When deemed appropriate, an estimation 28-1 of the later refractive error is then inferred during a first later period T1 from the later visual acuity measurements 26-1 and the associated later blur data 27-1. A similar process is repeated during a subsequent period T2, leading to infer the later Rx estimation 28-2 from the later visual acuity measurements 26-2 and the associated later blur data 27-2.

VA measurements may be based on any of multiple kinds of optotypes, as well known to the skilled person. Depending on the implementations, they may e.g. use a Snellen chart, LogMAR-ETDRS chart, Landolt C chart, Tumbling E chart, Golovin-Sivtsev table. In some modes, exploited optotypes include at least one of a circle, a square, a triangle, and a vanishing optotype with circles and/or lines, a grey background and/or a checkboard background.

The VA measurements may rely on optical instruments. They may rely on computerized tests enabling self-administered VA measurements. In the latter case, VA may be assessed by an adaptive staircase method such as the best-PEST procedure (for "best Parameter Estimation by Sequential Testing"), and more particularly by the FrACT method (for "Freiburg Visual Acuity and Contrast Test"), as described by M. Bach in "The Freiburg Visual Acuity Test - Automatic Measurement of Visual Acuity", Optom. Vis. Sci., vol. 73, pp. 49-53, 1996.

In particular modes, VA testing includes roughly determining the VA of the user's eye with a scale and then with use of shorter staircase.

In some modes, which may be combined with the previous ones, the VA is determined at a more accurate level than a line level in an eye chart (binary test for each line), by considering a percentage of good answers at a given level, e.g. 60 %. For instance, a VA of 1.25 corresponds to the ability to give 60 % of good answers with optotypes where details are seen angularly at 1/1.25 arcmin.

In some implementations, VA is measured by relying on a parametric probabilistic model of VA response and on artificial intelligence, as described e.g. by C. Piech et al. in "The Stanford Acuity Test: A Precise Vision Test Using Bayesian Techniques and a Discovery in Human Visual Response", Proceedings of the AAAI Conference on Artificial Intelligence, 34(01), pp. 471-479, 2020.

Since in setting the specific model 24 by the device 1, two or more VA measurements (corresponding to distinct levels of blur) are performed in association with the respective reference blur data 22, the VA testing is selected so as to achieve a good balance between test duration and accuracy.

With the device 1B or 1C, the type of VA testing performed during the reference period T0 for setting the specific model 24 (or a subsequent period exploited for updating or refining the specific model 24) may differ from the type of VA testing executed after that period T0 by the device 1C or 3 for inferring the Rx estimation 28. For example, the VA testing during T0 may be less complex than during later periods, so as to reduce the time required for the user for the multiple VA evaluations corresponding to the different levels of blur. On the contrary, the VA testing during T0 may be more complex than during later periods, so as to precisely set the parameter values 25B.

The broad range of working capacities of the device 1 will be exemplified below in various implementations.

Examples 1 - Refractive error estimation from VA at multiple distances

In the present modes, corresponding to particular version of the device 1A, the VA is estimated at two or more distances from the user's eye (thereby constituting the reference VA measurements 21 associated with selected distances as the reference blur data 22), an individual model is built (construction of the complete specific model 24) and the Rx estimation 25A is deduced from the collected data.

Distance monitoring and VA testing may be effected by any available method. They may notably be performed entirely by means of a mobile device, including a front camera and a screen, and of a mirror, as described in above-cited patent application WO 2022/013410 to Essilor International. According to such implementations, the user is positioning himself/herself in front of the mirror at a distance d/2, the mobile device is positioned with the front camera facing the mirror as close as possible to the head of the user and covering the considered user's eye, the distance d between the front camera and a virtual image of the mobile device in the mirror is measured, the screen of the mobile device displays optotypes of suited shapes, positioning and dimensions, and the VA is evaluated. As developed in that document, the distance d may be measured by displaying on the screen of the mobile device one or more elements (e.g. optotypes) having known dimensions, and by estimating the distance d based on the angular resolution of the front camera.

Alternatively, distance monitoring may be carried out by a time-of-flight camera available in the mobile device, e.g. an infrared camera. The monitoring may be obtained by a 3D sensor involving a depth map, and possibly exploit images from the front camera in combination.

In variants, distance monitoring by a mobile device is based on accelerators and gyroscopes, such as by exploiting an IMU (Inertial Measurement Unit) integrated in the device. A proper initial calibration is then desirable, which may be done e.g. by fitting a viewed remote object (e.g. a card) having a known dimension to a specific size on screen, corresponding to a predefined distance (e.g. 1 meter).

In still other distance monitoring implementations, in which user position is assessed by a remote device, distance is estimated by exploiting visible user physical features. For example, subject to a proper preliminary calibration step, one or two of the user's eye widths or the interpupillary distance can serve as reference dimension units.

In some modes combining two kinds of VA monitoring by a mobile device, VA tests are effected directly with the mobile device below 50 cm from the user's eye, and are exploiting a mirror as mentioned above for a distance over 50 cm. In advantageous implementations, this may enable measurements between 0.2 and 4 meters with a few percent accuracy.

In alternative execution modes, instead of involving a mirror, VA tests are carried out for the user's eye with the support of another person. The latter may e.g. hold, at a proper distance to the user, a mobile device on the screen of which proper optotypes are displayed.

In still other modes dispensing with a mirror as well as another person, a mobile device on the screen of which optotypes can be displayed is provided with remote control capabilities, e.g. based on voice or gesture recognition, and with recording capacities. The user may then position the mobile phone at an appropriate place and if possible arranged vertically, and stand in front of and at desired distances to the screen while giving instructions, e.g. requesting to indicate the distance to the user's eye, to display selected optotypes (shapes, sizes, orientations, etc.), and to take account of and record user's feedbacks associated with respective test items.

In the absence of astigmatism or if it can be neglected, the refractive error of the user's eye may be represented by a spherical defocus S (expressed in dioptres, as a need of correction). This entails a defocus blur $\delta$ that can be formulated as follows in function of accommodation A (expressed in dioptres), taking values between 0 and $A_{max}$, of distance *Dist* to the user's eye (positive, expressed in meters), and of S:

$$\delta = |\, S - A + (1\,/\,Dist)\,| \tag{1}$$

The accommodation A depends itself on the spherical defocus S and distance *Dist.* Noting:

$$S' = S + 1/Dist \tag{2}$$

we have:

$$\delta = |\, S' - A \,| \tag{3}$$

$$\begin{cases} A = 0 \; if \; S' \leq 0 \\ A = S' \, if \; 0 < S' < A_{max} \\ A = A_{max} \; if \; S' \geq A_{max} \end{cases} \tag{4}$$

Namely, A is worth its maximum value $A_{max}$ below the punctum proximum (PP), decreases linearly with the reciprocal of the distance between the PP and the punctum remotum (PR) down to zero, and remains constantly null beyond the PR. Also, the distances corresponding to the PR and the PP are respectively worth -1/S and - 1/($S - A_{max}$).

The visual acuity VA can be expressed in function of the defocus blur $\delta$, i.e. in function of the spherical defocus S, distance *Dist,* and accommodation A, and hence in function of S, *Dist* and $A_{max}$ (equations (2), (3), (4)).

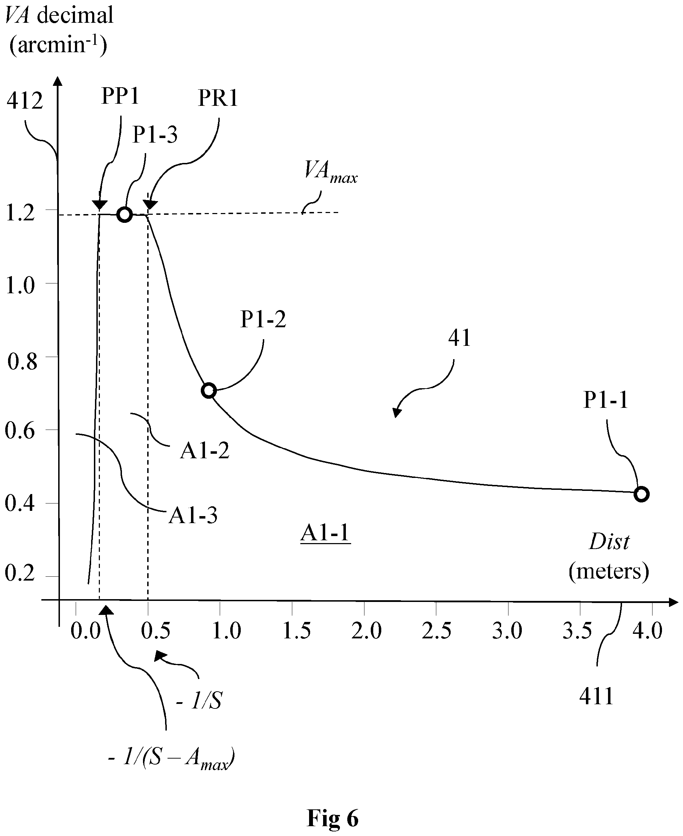

This is illustrated on **Figure 6** for a specific myopia case in which the user's eye has a defocus error of -2D (spherical defocus S for myopia) and a maximum accommodation $A_{max}$ equal to 4D. The curve 41 giving theoretical decimal VA (in arcmin$^{-1}$, axis 412) in function of the distance *Dist* (in meters, axis 411) has three areas: a far vision area

14

A1-1 beyond the PR, noted PR1, on which A is null or supposed so (it could be stimulated by other factors); an accommodation area A1-2 between PR1 and the PP, noted PP1, on which the defocus blur $\delta$ is supposed to be null; and a near vision area A 1-3 from the user's eye to PP1, on which A is worth $A_{max}$. In the considered example case, PR1 and PP1 are worth respectively 0.5 m (i.e. - 1/S) and 0.17 m (i.e. - 1/(S - $A_{max}$)). Moving from the maximum represented distance to the user's eye: in the far vision area A1-1, the VA increases with *Dist* varying from 4 m to 0.5 m as the defocus is reduced; on the accommodation area A1-2, the VA reaches its maximum $VA_{max}$ (and the MAR accordingly reaches its minimum $MAR_{min}$) while the accommodation A increases from 0 to $A_{max}$; below 0.17 m, the VA decreases, as the accommodation A cannot compensate for the defocus generated by the short distance.

The generic model 23 can be based on the previously cited Gómez-Pedrero article. In this respect, the VA can be expressed as:

$$VA = \frac{1}{[E_1 + E_2 \delta^q]^{1/q}} = \frac{1}{[E_1 + E_2(|S - A + 1/Dist|)^q]^{1/q}} \qquad (5)$$

where $E_1$ and $E_2$ are parameters depending on a pupil diameter D of the user's eye and being specific to each subject.

The parameter $E_1$ can be expressed in function of $VA_{max}$ by:

$$E_1 = VA_{max}^{-q} \qquad (6)$$

This results from equation (5) on the accommodation area A1-2, on which the defocus blur $\delta$ is null and the VA is constantly worth $VA_{max}$.

Consequently, for the far vision area A1-1 in which accommodation A is null:

$$VA = [VA_{max}^{-q} + E_2 |S + 1/Dist|^q]^{-1/q} \qquad \text{when } Dist \geq PR1 \qquad (7)$$

This can be formulated with the MAR as:

$$MAR = [MAR_{min}^q + E_2 |S + 1/Dist|^q]^{1/q} \qquad \text{when } Dist \geq PR1 \qquad (8)$$

$$MAR = MAR_{min} \qquad \text{when } PP1 < Dist < PR1 \qquad (9)$$

In this theoretical model, the same formulae (7) and (8) stand also true in the near vision area A1-3, subject to replacing S with (S - $A_{max}$) therein. Insofar as myopia is concerned, however, the device 1A may focus on operations in the far vision area A1-1 and the accommodation area A1-2.

The generic model 23 thus relies on 5 unknown variables: the spherical defocus S (in dioptres), *Dist* (in meters), $VA_{max}$ (in arcmin$^{-1}$), q (without unit) and $E_2$ (in m$^q$.arcmin$^q$).

As developed in the Gómez-Pedrero article, parameter q amounts to a tolerance to defocus. It is typically comprised between 1 and 3, and generally between 1.8 and 2.

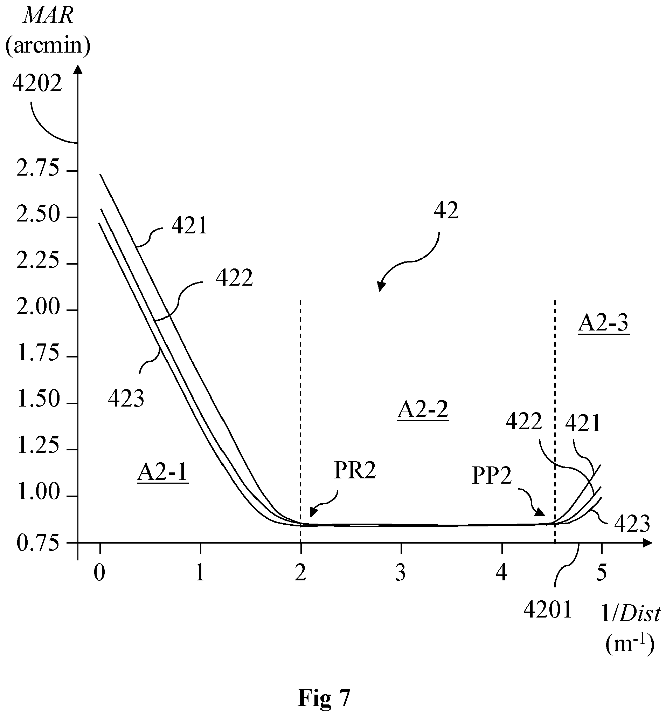

The role of parameter q is e.g. illustrated on example curves 42 of **Figure** 7, on which the MAR is represented (in arcmin, axis 4202) in function of 1/*Dist* (in m$^{-1}$, axis 4201) for a myopia of -2D and different values of q, being respectively worth 1.5, 2 and 2.5 for curves 421, 422 and 423. As apparent, a same PR corresponding to 2 m$^{-1}$, noted PR2, and a same PP (corresponding to around 4.5 m$^{-1}$), noted PP2, are obtained for the three curves 421, 422 and 423, which are constantly at the $MAR_{min}$ level on an accommodation area A2-2 between PR2 and PP2. However, in a far vision area A2-1 beyond PR2 and a near vision area A2-3 below PP2, the MAR variation slopes increase with decreasing values of q.

In the present embodiments, the device 1A is configured for evaluating the spherical defocus S and other required parameter(s) from VA measurements at different properly chosen distances. In this respect, the parameter q may be approximated to a fixed value, e.g. 2, for the sake of simplicity. It is then possible, on the ground of equation (7), to calculate S, $E_2$ and $VA_{max}$ from 3 pairs of distances *Dist* and associated measured VA.

In modes relevant to myopia, one of the pairs *(Dist, VA)* corresponds to the flat accommodation area and two other pairs to the far vision area. For example, as illustrated on **Figure 6,** points P1-1 and P1-2 are located in area A1-1, while point P1-3 is located in area A1-2. The device 1A may then extract $VA_{max}$ from point P1-3, and thereby infer *S* and $E_2$ from P1-1 and P1-2.

The maximum VA in the accommodation area A1-2 may be measured directly, so that only $S$ and $E_2$ need to be obtained from equation (7), which can be done via P1-1 and P1-2.

In variants, the three pairs *(Dist, VA)* are selected in the far vision area A1-1.

At any rate, the distances *Dist* exploited for specifying the generic model 23 through equation (7) deserve particular attention. Indeed, the accuracy of the obtained estimations depend on the accuracy of the pairs *(Dist, VA),* but also on their positioning on the VA curves.

More than 3 distances *Dist,* e.g. 4 distances, may be exploited in assessing the three unknown parameters of equation (7). Though redundant, those operations may enhance the precision and reliability of the results.

In variants, the device 1A is configured for determining also the parameter q in addition to $VA_{max}$, S and $E_2$, from at least 4 pairs *(Dist, VA).* Though being more demanding, such implementations may enhance the resulting precision.

In particular implementations facilitating the selection of the computation distances *Dist,* a backward mechanism is iteratively executed by the device 1A enabling the obtained results to be exploited by reinjecting them for further distance selection (as the inferred reference blur data 22').

Such implementations may possibly be initialized by a coarse estimation of the refractive error. For example, the initial estimation may be based on the Swaine rule, according to which the spherical defocus of a myopic eye can in a certain VA range (usually between 1/10 and 5/10) be evaluated from far vision VA (expressed in tenths), by dividing 0.25 by the latter.

An illustrative estimation scheme is given below for low to mid myopia (-2 D to -0.5 D), with q being e.g. set to 2 so that in far vision, from equation (8):

$$\mathrm{MAR}^2 = MAR_{min}{}^2 + E_2 \left| S + 1/Dist \right|^2 \qquad (10)$$

At an initial step, $MAR_{min}$ and $E_2$ are initialized at respective values $MAR_{min1}$ of 1 arcmin and $E_{2.1}$ of 1.5 $\mathrm{m}^2.\mathrm{arcmin}^2$ (for a pupil of 3 mm), and an initial far vision distance *Dist1* is selected, e.g. at 4 m. At that distance *Dist1,* VA is measured as equal to *VA1* (which may correspond to P1-1 on **Figure** 6) so that we get a first approximation $S_{est1}$ of the spherical defocus S:

$$S_{est1} = -\ 1/Dist1 - \sqrt{\frac{\left(\frac{1}{VA1}\right)^2 - (1')^2}{E_{2-1}}} \qquad (11)$$

The estimated spherical defocus $S_{est1}$ enables a first estimation $D_{estPR1}$ of the PR distance, equal to - $1/S_{est1}$, to be also obtained. Other starting points may be used instead of the present one, which may be suited to the specific user's eye condition, e.g. myopia level.

In a second step, a second distance *Dist2* is selected between $D_{est1}$ and *Dist1.* For example, it is given by an arithmetic average applied to the reciprocals of the distances:

$$1/Dist2 = 0.5\ (1/D_{estPR1} + 1/Dist1) \qquad (12)$$

which amounts to:

$$Dist2 = 2\ Dist1\ /\ (1 - S_{est1}.Dist1) \qquad (13)$$

With this second position, the visual acuity *VA2* can be measured (which may correspond to P1-2). The pair *(Dist2, VA2),* together with the previously obtained pair *(Dist1, VA1),* enable better assessments $S_{est2}$ and $E_{2-2}$ of respectively the spherical defocus S and parameter $E_2$ from equation (10). From $S_{est2}$, an enhanced estimated value $D_{estPR2}$ of the PR distance can be inferred.

In a third step, a third position *Dist3* is chosen in the estimated area of accommodation (sufficiently below $D_{estPR2}$, which may correspond to P1-3), so as to directly measure an estimated value $VA_{max3}$ or $MAR_{min3}$ of respectively $VA_{max}$ or $MAR_{min} = 1/VA_{max}$. Once $MAR_{min}$ is assessed, the previously obtained pairs *(Dist1, VA1)* and *(Dist2, VA2)* can be introduced into equation (10) as specified with the $MAR_{min3}$ value, so as to derive enhanced assessments $S_{est3}$ and $E_{2-3}$ of respectively the spherical defocus S and parameter $E_2$. An enhanced value $D_{estPR3}$ of the PR distance may also be deduced as equal to - $1/S_{est3}$.

The process may be stopped at that third step, taking $S_{est3}$ as the resulting estimation of the spherical defocus S (i.e. the Rx estimation 25A).

However, the process may alternatively be pursued with a fourth step, adapted to obtain an enhanced estimation of parameter q instead of 2. This may be done e.g. by selecting a fourth point ($Dist4$, VA4) between $D_{estPR3}$ and $D_{estPR2}$, and exploiting equation (8) together with the three pairs ($Dist1$, $VA1$), ($Dist2$, $VA2$) and ($Dist4$, $VA4$) so as to extract values $S_{est4}$, $E_{2-4}$ and $q_4$ of the respective three unknowns S, $E_2$ and q. This extraction may notably be performed through numerical optimization via an iterative method, as known to a skilled person - e.g. by means of a gradient descent applied to a cost function. The process may further include checking that the inferred value $D_{estPR4}$ = - $1/S_{est4}$ of the PR distance is above $Dist3$, used for measuring $VA_{max}$.

In a variant, it may be checked that the estimated maximum VA value, $VA_{max3}$, does not lie in the near vision area instead of the accommodation area. In this respect, a distance value lower than and sufficiently close to $Dist3$ may be selected, e.g. 0.90 $Dist3$, and it may be verified that the related VA measure provides a value close to $VA_{max3}$, thereby confirming the positioning in a flat area corresponding to the accommodation area.

In a refined version of the generic model 23, the defocus blur $\delta$ is released in the accommodation area A1-2 instead of being set constantly null, so as to represent a lag of accommodation. This enables under-accommodation of the user's eye to be integrated with respect to a requested stimulus, which is known to happen even if that stimulus is below accommodation capabilities, thereby slightly increasing VA loss.

In a related example implementation, a "lag term" inversely proportional to the distance $Dist$ is added to the defocus blur $\delta$ (which may e.g. be inserted into formula (3) outside the absolute value). This term involves a multiplicative factor AG corresponding to an accommodation gain, e.g. set between 0 and 1.

In another implementation, a constant "dark focus" term $dkF$ is added to the defocus blur $\delta$ (which may e.g. be inserted into formula (3) outside the absolute value), which reflects a remaining accommodation in the absence of any stimulation, i.e. in the dark.

In a more refined implementation, the generic model 23 takes account of both aspects above, and involves thus the lag term AG / $Dist$ together with the dark focus term $dkF$. In addition, while considering the accommodation gain AG lying between 0 and 1, the dark focus term $dkF$ is weighted with the multiplying factor (1 - $AG$), so that it represents a neutral point without lag expressed as (1 - $AG$) $dkF$. Consequently, if AG is worth 0, no lag term is present and the dark focus term is added as such (the pure dark focus term implementation above). By contrast, if $AG$ is worth 1, no dark focus term is present and the lag term is equal to 1 / $Dist$ (maximum lag representation). The generic model 23 may be more realistic by choosing a value of the accommodation gain $AG$ lying between 0 and 1 and sufficiently remote from 0 and 1, thereby generally leading to the added term: $AG$ / $Dist$ + (1 - $AG$) $dkF$. In advantageous modes, the accommodation gain $AG$ and/or the dark focus term $dkF$ are processed as further parameters, which may be estimated a *priori* and/or derived from proper measurements and computations in a way similar to other parameters.

The above examples are directed to myopia and focused on the far vision area and the accommodation area. As apparent to the skilled person, similar operations may be instead executed in the near vision area as well, which could be particularly relevant for eye conditions such as notably hyperopia or presbyopia.

In particular, the pairs ($Dist$, VA) may be selected in the near vision area and accommodation area, instead of the far vision area and accommodation area.

In variants, the device 1A is configured for selecting the pairs ($Dist$, VA) by jointly exploiting at least one point in the far vision area and at least one point in the near vision area in inferring the unknown parameters from equation (7).

Also, the device 1A may be adapted to execute two or more of the above implementations, in any possible combination.

Parameter initialization in an iterative process and/or partial parameter setting, which enables one or more model parameter to be set upstream, can be possibly based on previously estimated or monitored values. In this respect, a preliminary version of the (partial or complete) specific model 24 linking VA to refractive error may notably be built on the ground of machine learning and/or data gathering. Such a preliminary model may be fed with personalized parameters, including at least one of age, ametropia, gender, and ethnicity. The preliminary model may further or instead be adapted in function of a population to which the user belongs, as retrieved from database information. Such a population may e.g. be delineated as a subgroup of ametropic persons having similar current refraction and/or similar current visual acuity, or a subgroup of presbyopic persons having similar age and/or similar corrective addition. The preliminary model may further or instead be based and/or refined on the ground of data gathered about the specific considered individual user, thereby directing and/or refining predictions with respect to that user.

In addition, parameter initialization may be at least partially based on values in a prescription for a user already wearing eye glasses. Notably, the VA in far vision may give a good starting point for $VA_{max}$.

A user wearing glasses may further keep them in the measurements, so that a refractive error offset be evaluated instead of the complete value. Regarding myopia, this may be relevant where the residual defect is large enough, typically above 0.25 D at 4 m. As concerns presbyopia, however, even smaller values may be suited to differential estimations.

Examples 2 - Model parameter(s) from VA at multiple distances

In the present modes, instead of, or in addition to, providing the Rx estimation 25A, the device 1 (device 1B or 1C) is configured for providing the parameter values 25B partially or completely determining the specific model 24 during the reference period T0. This can then be exploited for deriving updated estimations of the refractive error during later periods.

The established specific model 24 may be complete, in which case particular values of the distance *Dist* and of the pupil diameter *D* are notably fixed (e.g. respectively 6 meters and 3 mm), or it may be partial, leeway being then left to at least part of the involved parameters (e.g. *Dist* and *D*, thus $VA_{max}$ and $E_2$, but not q).

For instance, coming back to cases of Examples 1, not only the spherical defocus *S* but also $VA_{max}$, q and $E_2$ are evaluated together with *S*. In particular implementations, at least one of the assessed parameter values is kept for later estimations of the refractive error. Then, this can be reused in later periods without requiring re-computation, thereby alleviating the related operations. For example, q and possibly $E_2$ may be considered significantly more stable over time than *S* and $VA_{max}$, and thus kept for substantially longer than the period T0. That duration may further depend on the nature of the considered parameter, and e.g. be different for q and for $E_2$.

The distance monitoring and VA evaluations may be carried out with a mobile device as mentioned above within Examples 1, for setting the specific model 24 via the parameter values 25B during period T0 as well as for exploiting those values 25B in later Rx estimations.

In other embodiments, in particular when the parameter values 25B, excluding the distance *Dist,* are assessed by an ECP in dedicated premises during the period T0, the distances are e.g. measured by a dedicated remote monitoring apparatus, or directly available on the floor or on a wall in the form of graduated strips. This amounts to a kind of upstream calibration. The later assessment of the refractive error based on those parameter values 25B may then exploit a mere mobile device for distance monitoring and VA evaluation as previously described.

In some implementations directed to assessment of the parameter values 25B, an ECP provides the user with a trial frame lens having an aimed correction, and makes also available to him/her an application (e.g. on mobile phone) adapted to conduct first VA tests in calibration mode. In other implementations, the user is provided with adapted trial lenses for effecting such an evaluation, possibly under remote supervision by an ECP.

In other embodiments, the refractive error of the user's eye is measured during the reference period T0 and provided as input to the device 1B or 1C (it is then part of the reference blur data 22). That device is accordingly configured for providing the parameter values 25B, the refractive error being already known.

For instance, a triplet including the distance *Dist,* the VA and the spherical defocus *S* is obtained during period T0 from measurements in the far vision area. The unknown(s) among $VA_{max}$, $E_2$ and q may then be estimated by relying on equation (7). If e.g. q is set to 2, only $VA_{max}$ and $E_2$ need to be evaluated, so that measurements at two distances in the far vision area may be enough. If q is also an assessment target, the measurements at three or more distances are effected. A feedback loop mechanism described in the Examples 1 may be used.

Once all desired parameters have been assessed during the reference period T0, it is possible to rely on them at later stages in estimating the refractive error. For example, q and $E_2$ being kept during a preset duration (e.g. between 1 and 3 months) substantially longer than T0 (e.g. 1 hour), only $VA_{max}$ needs to be estimated together with *S* in that duration. This reduces the number of unknowns from 4 to 2, solved from two or more pairs *(Dist, VA)* obtained from the later VA measurements 26 and later blur data 27. Such operations may be regularly repeated (e.g. once a week) all along the preset duration.

Alternatively, or in combination, the value of $VA_{max}$ estimated during the reference period T0 is kept during a duration longer than the reference period T0 but shorter than the preset duration used for $E_2$ and q. For example, T0 is worth 1 hour, weekly follow-up measurements are performed, $VA_{max}$ is kept unchanged for 1 month, and q and $E_2$ are kept unchanged for 3 months.

In variants, the accommodation area is regularly updated from estimations carried out after the period T0. When proceeding with a latest batch of measurement operations, it can thus be considered that the accommodation area is approximated at a degree sufficient for proceeding directly with one or more VA measurements at a relevant distance inside it, thereby deducing $VA_{max}$. The overall required measurements and/or computations may thus be significantly alleviated.

Like with Examples 1, while measurements in the far vision area are particularly suited to myopia, measurements may also or instead be carried out in the near vision area, in particular in case of other eye conditions such as notably hyperopia or presbyopia.

Examples 3 - Model parameter(s) from VA with one or more optical power corrections

In the present modes, again, the device 1 (device 1B or 1C) is configured for providing the parameter values 25B partially or completely determining the specific model 24 during the reference period T0. This can then be exploited for deriving updated estimations of the refractive error during later periods.

However, instead of obtaining VA measurements for different distances, the measurements are effected for different optical power shifts, a zero optical power shift (i.e. no optical power correction) being possibly included among the measurements.

For example, the generic model 23 is expressed by the simplified formula:

$$S = k * VA + l \qquad (14)$$

where VA is the visual acuity measured at a predetermined distance from the user's eye (e.g. at 6 meters or 20 feet), and $k$ and $l$ are parameters. From formula (14), a resulting differential evolution model is given by:

$$\Delta S = k * \Delta VA \qquad (15)$$

where "$\Delta$" stands for a variation over time, applied to the spherical defocus $S$ or to the visual acuity $VA$.

In particular implementations, that evolution of the spherical defocus S is equated to an Rx variation imposed by optical power correction during the reference period T0, the resulting VA variation during T0 being equated with the VA variation over time. In this respect, a lens is arranged in front of the user's eye so as to cause a desired optical power correction corresponding to a variation $DiffS1$ of $S$, for example +0.25 D or +0.5 D for a myopic eye. The corresponding VA variation $DiffA1$ is then measured. From equation (15), the parameter $k$ can thus be estimated as the ratio $DiffS1 / DiffA1$.

After the reference period T0, the device 1C or 3 is configured for inferring from the measured VA variation $\Delta VA$ and formula (15), the variation $\Delta S$, and hence an estimation of the refractive error S.

Though such an estimation can only provide a coarse approximation of Rx evolutions, it may offer an interesting first evaluation during a substantial period of time subsequent to the reference period T0. In particular, it may enable a system or a person to identify a critical situation calling for a recommendation such as e.g. making an appointment with an ECP. This may notably be exploited in the follow-up of myopia progression.

In fact, the simplified generic model 23 expressed through formula (15) may be deemed satisfying as long as no substantial Rx evolution takes place (which may be quantified by a relative threshold, e.g. 10 % or 20 % variation).

This approximation may also be used for initializing an iterative process compliant with feedback methods described in the Examples 1 or Examples 2, so as to obtain more precise and relevant estimations.

In addition, the refractive error and VA measurements may be performed by an ECP with specialized equipment, so as to provide an accurate estimation of the $k$ ratio parameter. After the reference period T0, the refractive error may be straightforwardly derived from VA measurements, possibly by a simple mobile apparatus.

While using a single power correction lens may be enough for specifying the model associated with equation (14) (based on VA measurements respectively with and without that lens), in advantageous embodiments, two or more lenses are used, e.g. 0.25 D and 0.5 D. In this way, the parameter $k$ can be estimated with better precision (since relying on three or more points instead of two), e.g. through linear regression.

In those implementations, particular care can be taken to proceed with such calibration measurements at very close moments in similar or same environment, so as to turn down prejudicial effects of acuity change during the day or versus light condition.

Though the refractive error has been expressed above by the spherical defocus S, alternative or further Rx components may be processed in a similar way.

Also, a more complex model than the linear one may be used, e.g. compliant with equation (7), the proper number of optical power corrections being imposed for determining the unknowns.

The present implementations may be combined with the consideration of multiple values of the distance $Dist$ as disclosed in Examples 1 and 2.

The exploitation of one or more power correction lenses may also be particularly attractive in measuring other conditions than myopia. Notably, in particular implementations, the device 1 is used for extracting the information 25 relevant to the refractive error of a hyperopic eye (whether the estimation itself 25A or the model parameters 25B). This may be particularly relevant for the user being aged less than 35 years, for which presbyopia is normally not a preponderant cause of blur at short distances. In related modes, at least part of the reference VA measurements 21 is carried out beyond the PP while imposing a positive power lens on the user's eye. This approach makes possible to mimic a myopic behaviour in spite of considering a distance normally associated with a correct vision and a maximum VA, the latter being purposely deteriorated by the artificially altered optical power. This amounts to generating a PR and a far-vision area beyond it, previously absent for the hyperopic eye, in addition to making the PP closer to the user's eye and to accordingly modifying (i.e. bringing closer and reducing) the near-vision and accommodation areas.

In particular implementations, it is proceeded as developed in Examples 1 and 2, by measuring VA at multiple distances and inferring the information 25. A type of the generic model 23 as described above may notably be used, involving formula (5). Also, at least one of the selected points is located in the accommodation area in association with respectively one or more converging lenses imposed on the user's eye.

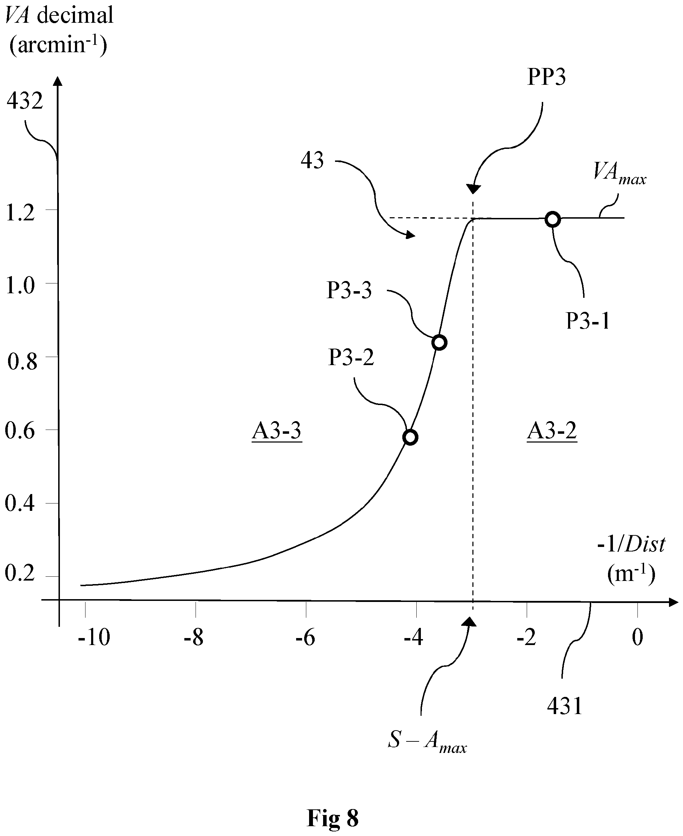

For example, as illustrated on **Figure 8** representing an evolution curve 43 of VA (axis 432, in arcmin$^{-1}$) in function of -1/*Dist* (in m$^{-1}$, axis 431) in a particular hyperopia case with $S$ = +3 D and $A_{max}$ = 6 D, the punctum proximum PP3 is worth about 33 cm (-1/*Dist* being worth $S - A_{max}$ = -3 m$^{-1}$) and separates a near-vision area A3-3 from an accommodation area A3-2. The VA of the user is regularly improving when moving away from the user's eye to PP3, where it reaches its maximum level $VA_{max}$ that is maintained beyond PP3 thanks to proper accommodation. More precisely, the accommodation A then fully compensates for the refractive error S, taking account of the distance *Dist* ($A$ = $S$ + 1/*Dist*).

For sake of comparison, an emmetropic eye (no refractive error, thus $S$ = 0) with $A_{max}$ being e.g. worth 8 D leads to a curve shape similar to the previous one, but with the PP located at 12.5 cm (-1/*Dist* being worth $-A_{max}$ = -8 m$^{-1}$). In addition, a presbyopic eye deprived of refractive error ($S$ = 0) and with $A_{max}$ being e.g. worth 3 D also leads to a shape similar to the previous one, but with the PP located at about 33 cm (-1/*Dist* being worth $-A_{max}$ = -3 m$^{-1}$). It deserves noting that the VA evolution is then hardly distinguishable from curve 43, so that the presbyopia and the hyperopia have in practice quite similar effects on the user's eye VA, though relying on different mechanisms, i.e. insufficient refractive power of the user's eye for hyperopia and deficient accommodation capacity for presbyopia.

Three points at respective distances to the user's eye are e.g. considered: P3-2 and P3-3 in the near-vision area A3-3, and P3-1 in the accommodation area A3-2. Point P3-1 is at a significantly more remote distance to the user's eye than PP3, and is subject to two distinct measurements: one without additional lens (eye naked or with usual correction, depending on the execution mode) and another with an imposed convergence power *DiffS3*, which is e.g. comprised between +0.25 D and +4 D, or more precisely between +0.5 D and +2 D. Multiple lenses having respectively distinct optical powers may further be successively imposed on the user's eye. The P3-1 measurement without additional lens can be used for directly extracting $VA_{max}$. On the other hand, the P3-1 measurement involving the additional lens may ensure to provide a measurement in a far-vision area beyond the accommodation area in case of hyperopia, and to distinguish it from emmetropia and presbyopia.

Though P3-2 and P3-3 measurements may provide useful VA information, in variants, only one of them or none of them is exploited, so that the test operations are alleviated by focusing on the accommodation area A3-2. In other embodiments, which may be combined with the previous ones, the P3-1 measurement(s) is/are carried out only by involving one or more lenses, or on the contrary only without lens. In the latter case, hyperopia may be distinguished from presbyopia by relying on the user age.

Examples 4 - Refractive error estimation from VA at one or more direction angles

The present embodiments are particularly suited to taking account of astigmatism. The refractive error of the user's eye can then be represented by the spherical defocus $S$, together with an axis component $\alpha$ (along which the refractive power is worth S) and a cylindrical defocus $C$. Also, a spherical equivalent $H$ of the user's eye may be given by:

$$H = S + C/2 \qquad\qquad (16)$$

The minus cylinder notation will be adopted below, so that the lowest refraction power $S_{min}$ and the highest refraction power $S_{max}$ are respectively worth:

$$S_{min} = S + C, \qquad S_{max} = S \qquad\qquad (17)$$

In particular implementations, the generic model 23 exploited by the device 1 is a more sophisticated version of the model developed in the Examples 1 and corresponding to equations (1) to (5), while taking the cylinder defocus $C$ into account. Thus, by noting:

$$H' = S + C/2 + 1/Dist \qquad\qquad (18)$$

the defocus blur $\delta$ in the generic model 23 is worth:

$$\delta' = \tfrac{1}{2}\sqrt{4\,(H' - A)^2 + C^2} \tag{19}$$

with:

$$\begin{cases} A = 0 & if\ H' \le 0 \\ A = H' & if\ 0 < H' < A_{max} \\ A = A_{max} & if\ H' \ge A_{max} \end{cases} \tag{20}$$

In formulae (20), $0 < H' < A_{max}$ corresponds to an accommodation area, and in the case of myopia (S < 0), H' $\le$ 0 and H' $\ge A_{max}$ correspond respectively to a far vision area and to a near vision area. The PR is given from formulae (18) and (20) by - 2 / (2S + C).

Two meridians are particularly considered in the generic model 23: one perpendicular to the axis component $\alpha$ and associated with the lowest refractive power $S_{min}$, corresponding to a defocus blur $\delta_1$, visual acuity $VA_{axis1}$ and MAR $MAR_{axis1}$, and another along the axis component $\alpha$ and associated with the highest refractive power $S_{max}$, corresponding to a defocus blur $\delta_2$, visual acuity $VA_{axis2}$ and MAR $MAR_{axis2}$.

The defocus blurs $\delta_1$ and $\delta_2$ are worth:

$$\delta_1 = |\ S_{min} + 1/Dist - A\ | = |\ S + C + 1/Dist - A\ | \tag{21}$$

$$\delta_2 = |\ S_{max} + 1/Dist - A\ | = |\ S + 1/Dist - A\ | \tag{22}$$

the defocus blur $\delta'$ being linked to defocus blurs $\delta_1$ and $\delta_2$ by the relation:

$$\delta' = \sqrt{\frac{\delta_1{}^2 + \delta_2{}^2}{2}} \tag{23}$$

(i.e. the root mean square radius of the ellipse of the blur).

In particular, in the accommodation area (formulae (19), (21) and (22) taking into account formulae (18) and (20)):

$$\delta' = \delta_1 = \delta_2 = |\ C / 2\ | \tag{24}$$

In what follows, the terms "mean MAR" are presently designating the MAR of the astigmatic user's eye corresponding to an equivalent sphere, i.e. which can be obtained by measuring MAR with a non-oriented test target. The same remark stands for "mean VA". In a manner similar to formula (5), the mean MAR, noted $MAR_{seq}$ and associated with the mean VA $VA_{seq}$, can be expressed as:

$$\begin{aligned} MAR_{Seq} &= \left[ MAR_0{}^q + Q.\delta'^q \right]^{\frac{1}{q}} \\ &= \left[ MAR_0{}^q + Q\left( \tfrac{1}{2}\sqrt{4\,(H'-A)^2 + C^2} \right)^q \right]^{1/q} \end{aligned} \tag{25}$$

where $MAR_0$, Q and q are model parameters.

That model can be derived from the developments made in the Gómez-Pedrero article (notably formula (8) therein), by introducing the further effect of the distance *Dist.*

$MAR_0$ corresponds to the best corrected VA for a given pupil size. The parameter q is similar to the one considered in the generic model 23 of Examples 1, reflects a tolerance to defocus, and is usually comprised between 1 and 3. Regarding parameter Q, it may be expressed in the generic model 23 according to the works in the Gómez-Pedrero article as equal to:

$$Q = (K.D)^q \qquad (26)$$

where $D$ still refers to the pupil diameter of the user's eye, $K$ being a further parameter.

Consistently, $MAR_{axis1}$ and $MAR_{axis2}$ are respectively expressed as:

$$MAR_{axis1} = \left[ MAR_0{}^q + Q \left| S + C + \frac{1}{Dist} - A \right|^q \right]^{1/q} \qquad (27)$$

$$MAR_{axis2} = \left[ MAR_0{}^q + Q \left| S + \frac{1}{Dist} - A \right|^q \right]^{1/q} \qquad (28)$$

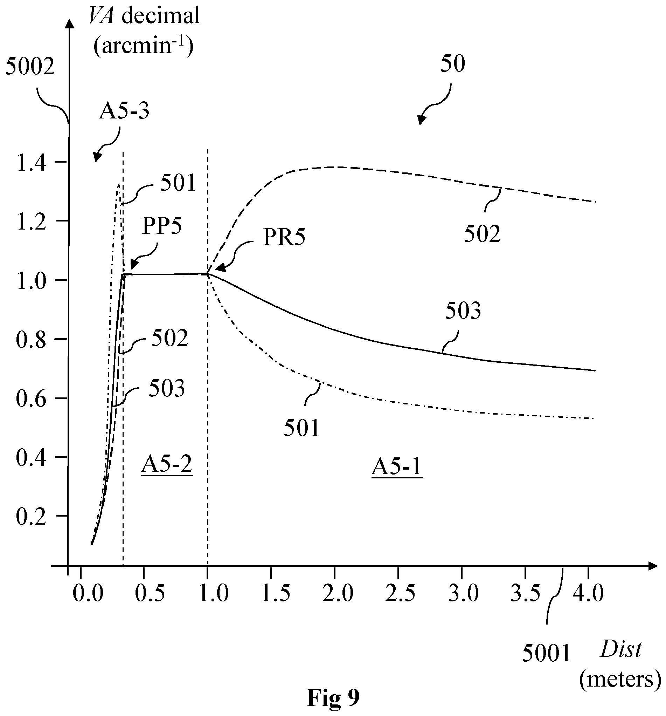

Illustrative trends of $VA_{Seq}$, $VA_{axis1}$ and $VA_{axis2}$ are shown with curves 50 of **Figure 9** representing VA (axis 5002, in arcmin$^{-1}$) in function of distance (axis 5001, in m) in the particular myopia case of $S = -0.5$ D, $C = -1$ D and $A_{max} = 2$ D. Then, the spherical equivalent is worth $H = -1$ D (formula (16)), delineating an accommodation area A5-2 between a PP of 1 m, noted PP5, and a PR of 0.33 m, noted PR5 (formulae (18) and (20)). The three curves 501, 502 and 503 corresponding respectively to $VA_{axis1}$, $VA_{axis2}$ and $VA_{seq}$ have a same scheme in the accommodation area A5-2, while differing significantly from one another in a far vision area A5-1 beyond PR5 and a near vision area A5-3 below PP5. The curve 501 ($VA_{axis1}$) has the lowest VA in far vision and best VA in near vision, the curve 502 ($VA_{axis2}$) has conversely the highest VA in far vision and lowest VA in near vision, and the curve 503 ($VA_{seq}$) is positioned between both.

Finally, the generic model 23 hinges on 7 parameters in determining VA: 3 for the refractive error, i.e. $S$, $C$ and $\alpha$; 1 for the distance $Dist$; and 3 corresponding to $MAR_0$, $Q$ and $q$. Exploiting $Dist$ as a variable providing the reference blur data 22, this leaves 6 unknowns to be assessed.

In some modes of the device 1, the resolution relies not only on variable distances but also on direction angles associated with one or more oriented test target. Accordingly, the reference blur data 22 include both distances and angles.

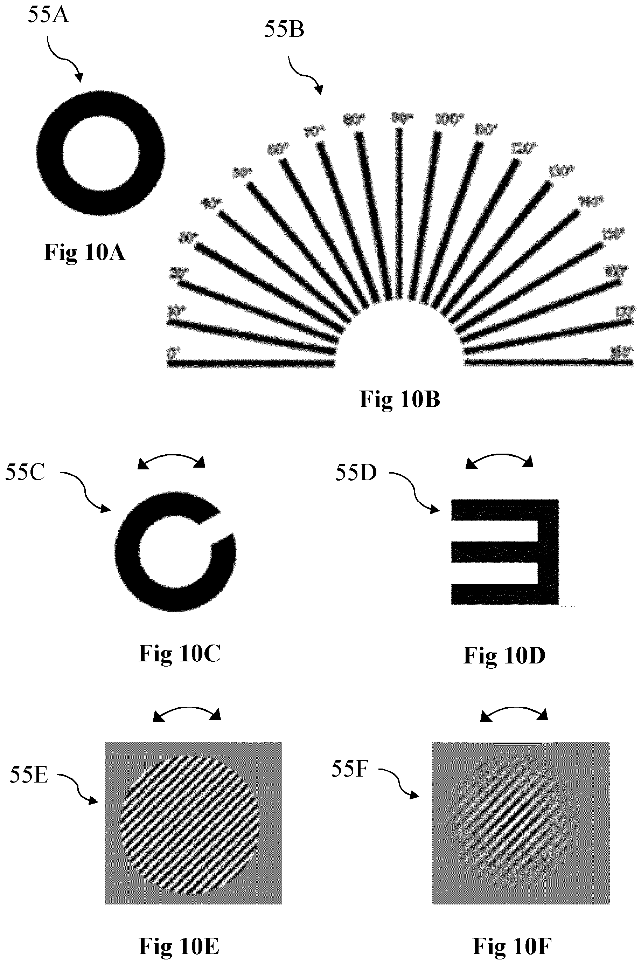

Particular related implementations include the following aspects:
Under a first strand, VA measurements of $VA_{seq}$ are performed at 3 or more different distances related to respectively three different levels of blur. It proves appropriate in this respect to use an optotype without specific orientation, as the one noted 55A represented on **Figure 10A** with a ring, particularly relevant to spherical equivalent determination. In a myopia case, the distances may include one in the accommodation area and two in the far vision area. An iterative process based on initial approximations may be used as developed with Examples 1.

Under a second strand, the axis component $\alpha$ is extracted. In some modes, this is done by a basic process using a Parent dial such as e.g. the one noted 55B represented on **Figure 10B,** displayed beyond the accommodation area of the user's eye (i.e. $A = 0$), as known to a skilled person.

In alternative modes, an oriented optotype is rotated in front of the user's eye beyond the accommodation area of the user's eye (i.e. A = 0), so as to achieve the maximum and/or the minimum of sharpness. This may be simply effected by displaying the optotype on the screen of a mobile device, e.g. a smartphone, and by turning the mobile device until reaching the relevant direction(s), and possibly identifying and recording the latter by mean of an IMU present in the mobile device (roll angle). The screen of the mobile device may be directed towards a mirror, so that the user determines the relevant direction(s) by looking at the optotype reflected in the mirror rather than directly on the screen. Larger distances between the user's eye and the visualized optotype can thus be obtained. The oriented optotype displayed on screen may e.g. take the form of, or include, an optotype 55C ("C") used in the Landolt C test or 55D ("E") used in the Tumbling E test (the latter having 4 possible orientations including upright, reversed, and tilted to the right or left), as represented respectively on **Figure 10C** and **Figure 10D.** It may instead take the form of, or include, a grating such as a disk of parallel lines, as represented on **Figure 10E,** possibly inside a Gabor patch, such as represented on **Figure 10F.**

According to variants, the optotype is rotated in the accommodation area of the user's eye.

Under a third strand, once the axis component $\alpha$ is extracted through the second strand, oriented optotypes are displayed to the user's eye at a determined distance beyond the accommodation area of the user's eye (i.e. $A = 0$), according to the astigmatism axis (axis component $\alpha$) and/or to the perpendicular axis ($\alpha + 90°$). The VA is then measured, yielding $VA_{axis1}$ and/or $VA_{axis2}$ at the selected distance $Dist$.

In advantageous modes, both $VA_{axis1}$ and $VA_{axis2}$ are measured. This can facilitate the computations, particularly for extracting $C$ as visible on formulae (27) and (28). In variants, only the astigmatism axis or only the perpendicular axis is used for the measurements with the oriented optotype, at two or more distances.

As will be apparent to the reader, while the third strand depends on the second strand, $\pi$ the first strand may be carried out in any order with respect to the second and third strands.

Alternatively, or in combination, VA measurements in one or more other directions than the astigmatism axis and perpendicular axis are used. With the generic model 23 developed above, the MAR $MAR(\theta)$ for an oriented optotype along an axis angle $\theta$ is given by:

$$MAR(\theta) = \left\{ MAR_0{}^q + Q \left( \left| S + C \sin^2(\alpha - \theta) + \frac{1}{Dist} - A \right| \right)^q \right\}^{1/q} \quad (29)$$

which relation can be used in evaluating the unknowns.

In particular modes, 3 measurements are carried out for the mean VA, including one in the accommodation area and two beyond, the axis component $\alpha$ is separately identified, and 2 measurements are carried out along respectively the found astigmatism axis and the perpendicular axis. This provides 5 independent equations from which estimations of the 5 unknowns $S, C, MAR_0, Q$ and $q$ can be inferred. The estimations may be effected by any proper digital iterative optimization method, as known to a skilled person.

As suggested above and visible on the formulae, multiple variant implementations are possible. For instance, all parameters except the axis component $\alpha$ (which can be determined independently) may be derived from mean VA measurements at various distances. In other examples, once the axis component $\alpha$ is known, all other parameters may be derived from VA measurements at various distances along a given direction angle. In other modes, VA measurements are carried out at three or more direction angles and at the same distance, including along the astigmatism axis and the perpendicular axis.

The remarks made about Examples 1 regarding parameter initialization and/or partial parameter setting are relevant here, too. Further, where such initializations are at least partly based on prescription values for a user having already eye-glasses, the indicated astigmatism angle may provide a good starting point for the axis component $\alpha$, in addition to the far vision correction for $VA_{max}$.

Also, in a similar way to the Examples 1 and Examples 2, the parameters may be fully or partly evaluated during the reference period T0, and may be fully or partly exploited in later VA measurements directed to evaluating the refractive error.

The embodiments of the present Examples 4 may further be combined with the exploitation of multiple optical power corrections, as described in Examples 3.


Examples 5 - Refractive error estimation integrating pupil diameter


In the above series of examples, the pupil diameter $D$ of the user's eye is at least implicitly taken into account by keeping same or similar user and environment conditions (notably luminosity) in proceeding with VA measurements. This is highly desirable because the pupil diameter $D$ is typically a first-order parameter in visual acuity models, the role of which is pointed out above and developed in the Gómez-Pedrero article.

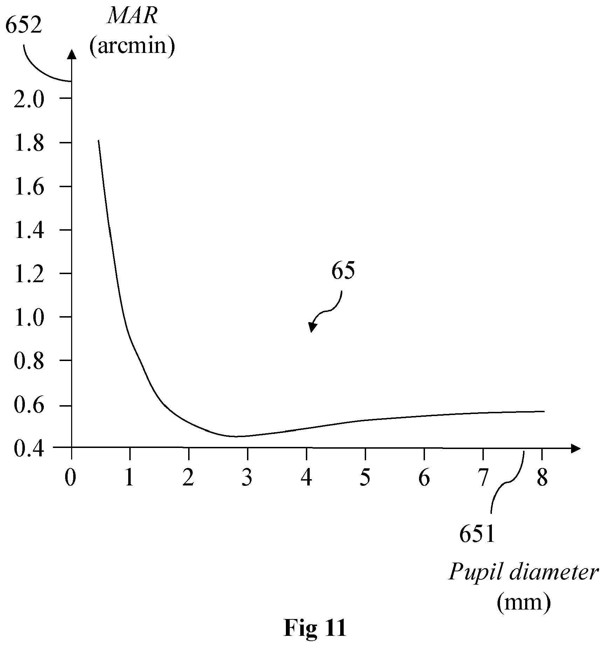

The influence of $D$ on $VA$ is illustrated on **Figure 11** schematically showing an evolution of the best corrected MAR (axis 652, in arcmin), i.e. the MAR without defocus blur (e.g. $MAR_{min}$ in Examples 1 and 2, $MAR_0$ in Examples 4, see formulae (5), (6), (25), (27), (28)), in function of the pupil diameter (axis 651, in mm) for a particular curve 65, and about which the reader can find detailed explanations in the Gómez-Pedrero article. As visible on the curve 65, for small values of $D$ up to a first size threshold of around 3 mm, the best corrected MAR is a strongly decreasing function of $D$ (diffraction profile limitation). Above that first size threshold, the best corrected MAR increases at a slow globally linear rate, due to the presence of high order aberrations (noted "HOA"). That increase remains effective up to a second size threshold of around 6 mm, above which the MAR stabilizes, which can be explained by the Stiles-Crawford effect, as known to the skilled person.

Accordingly, with low to no blur, the MAR varies in function of $D$ substantially as the best corrected MAR above. By contrast, for high enough amounts of blur (typically above 1 D), the MAR is approximately proportional to the pupil diameter $D$ and to the defocus blur ($\delta$, $\delta'$, $\delta_1$ or $\delta_2$, depending on the target measurements), while contributions of the best corrected MAR become weak of negligible.

All hereinafter implementations take account of the pupil diameter $D$ and can be notably combined with the Examples 1 to 4.

In some embodiments, the light conditions are carefully controlled.

In other embodiments, it is directly ensured that the pupil diameter $D$ of the user's eye is unchanged between different VA measurements. Such achievements can afford better precision, since even under same luminosity, $D$ may be subject to substantial variations over time and object distance.

Particular modes enabling such a pupil diameter control are exposed below, based on the use of a mobile device including at least a front camera for assessing $D$.

In near vision, typically up to a distance *Dist* of around 50 cm, *D* is directly measured with the camera of the mobile device. Relevant dimension information may be directly available via a dedicated mobile application. Alternatively, the pupil dimension may be inferred from a reference dimension, such as e.g. a distance between ears or an iris diameter. The latter relative estimation may prove quite reliable, because the iris diameter is very stable.

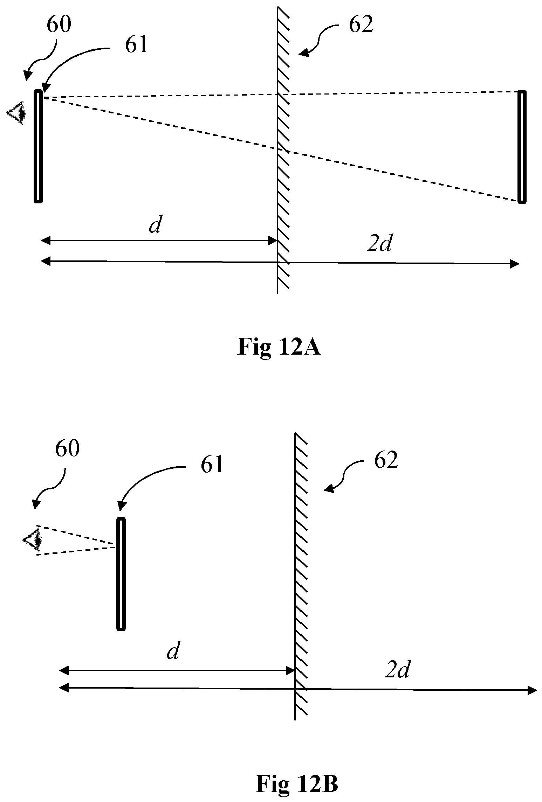

In far vision, typically at least 3 m away from the user's eye, a set-up similar to the one disclosed in the above-cited patent application WO 2022/013410 can be exploited. Namely, as illustrated on **Figures 12A and 12B** (not to scale), a mobile device 61 such as a smartphone provided with a front camera, a back camera and a screen is handheld by the user close to the user's eye 60, the screen of the mobile device is seen through a mirror 62 in front of the user and at a distance d from the mobile device, and the pupil diameter D is measured by the back camera. More precisely, the measurement process may be e.g. carried out with a smartphone as follows:

- the user positions himself/herself in front of the mirror 62;
- with the smartphone 61 kept close to the eye 60, the user adjusts the distance *Dist* as twice the distance *d* from the smartphone 61 to the mirror 62, typically between 3 and 4 m, using the front camera and a pattern or optotype displayed on the screen;
- keeping the selected position, the user moves the smartphone 61 away from the face, for instance at around 20 to 30 cm, and measures the pupil diameter *D* of the eye 60 with the back camera; like with near vision, the *D* variation may notably be assessed relative to the iris diameter of the eye 60;
- the surrounding lighting is adjusted until the desired pupil diameter *D* is observed;
- the user brings back the smartphone 61 close to the eye 60, thereby hiding the latter, and proceeds with a visual acuity test;
- the above operations are possibly repeated with different values of the distance *Dist*.

By controlling the lighting conditions, the same pupil diameter *D* can then be set in successive VA measurement operations.

In variants, the pupil diameter *D* differs between VA measurements instead of being kept stable, but is monitored and leads to variation compensation, on the ground of proper expression in the generic model 23. For instance, $VA_{max}$ and $E_2$ in equations (5) and (6) of the Examples 1 and 2, and $MAR_0$ and $Q$ in equations (25) to (29) of the Examples 4, are expressed in function of *D*. Related modelling representations can be found in the Gómez-Pedrero article, see notably related formulae (5), (6), (7) in that article.

Examples 6 - Refractive error estimation from VA at multiple pupil diameters

While the Examples 5 are taking account of the pupil diameter *D* in deriving Rx estimations from controlled variations of other entities, such as e.g. the distance *Dist* (Examples 1 and 2), optical power corrections (Examples 3) or direction angles (Examples 4), in the present embodiments, the pupil diameter *D* of the user's eye is itself directly exploited for imposing controlled variations enabling derivation of the Rx estimation 25A and/or the parameter values 25B. In practice, distinct values of *D* are part of the reference blur data 22 and associated with respective items of the reference VA measurements 21.

Again, relevant modelling representations can be found e.g. in the Gómez-Pedrero article.

Variations of *D* may be combined with variations of any or all of the other entities above.

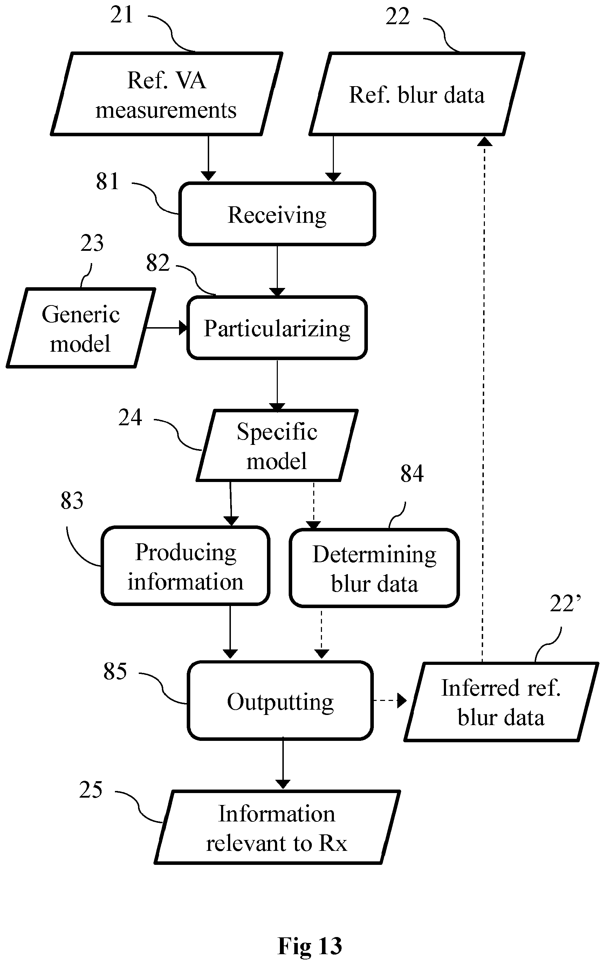

The process for estimating a refractive error of the user's eye from VA measurements is now detailed in an example potentially applicable to all above implementations, and possibly executed with the device 1. As represented on **Figure 13,** the process includes:

- receiving the reference VA measurements 21 and the associated reference blur data 22 (step 81);
- particularizing the generic model 23 to the specific model 24, from the received reference VA measurements 21 and reference blur data 22 (step 82);
- producing the information 25 relevant to refractive error (step 83);
- optionally, determining the inferred reference blur data 22' from the received reference VA measurements 21 and reference blur data 22 (step 84), and reinjecting those data 22' as further items of the reference blur data 22 to be exploited for further items of the reference VA measurements 21;
- providing the information 25 relevant to refractive error (step 85).

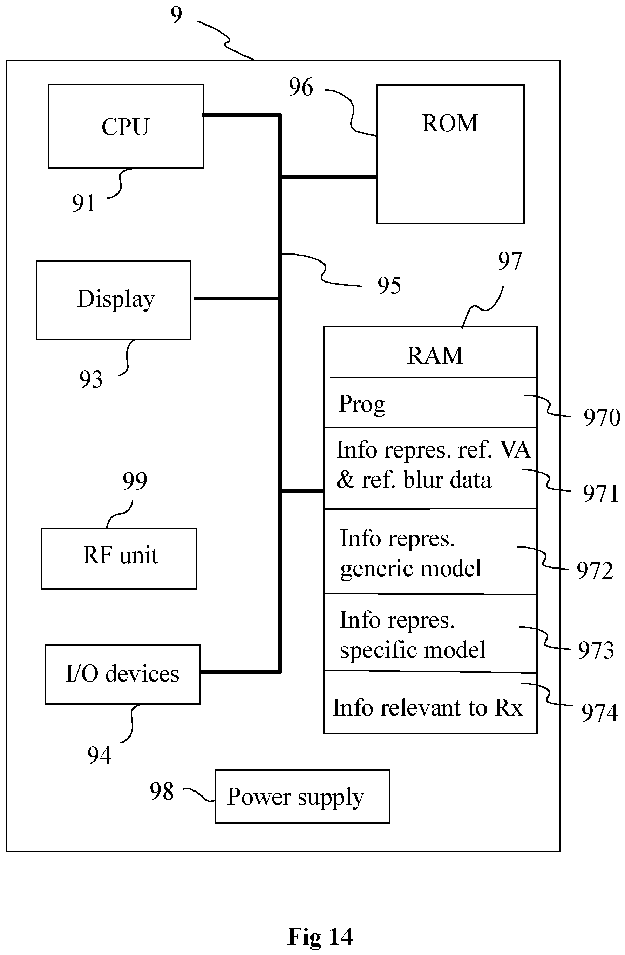

A particular apparatus 9, visible on **Figure 14,** is embodying the device 1 described above. It corresponds for example to a tablet, a smartphone, a head-mounted display (HMD), a laptop, a desktop computer or a workstation.

The apparatus 9 comprises the following elements, connected to each other by a bus 95 of addresses and data that also transports a clock signal:

- a microprocessor 91 (or CPU);
- a non-volatile memory of ROM type 96;
- a random access memory (RAM) 97;
- one or several I/O (Input/Output) devices 94 such as for example a keyboard, a mouse, a joystick, a webcam; other modes for introduction of commands such as for example vocal recognition are also possible;
- a power source 98; and
- a radiofrequency (RF) unit 99.

According to a variant, the power supply 98 is external to the apparatus 9.

The apparatus 9 also comprises a display device 93 of display screen type. According to a variant, the display device is external to apparatus 9 and is connected thereto by a cable or wirelessly for transmitting the display signals. The apparatus 9 comprises an interface for transmission or connection adapted to transmit a display signal to an external display means such as for example an LCD (for "Liquid-Crystal Display") or OLED (for "Organic Light-Emitting Diode") screen or a video-projector. In this respect, the RF unit 99 can be used for related wireless transmissions.

It is noted that the word "register" used in the description of memories 97 can designate in each of the memories mentioned, a memory zone of low capacity (some binary data) as well as a memory zone of large capacity (enabling a whole program to be stored or all or part of the data representative of data calculated or to be displayed). Also, the registers can be arranged and constituted in any manner, and each of them does not necessarily correspond to adjacent memory locations and can be distributed otherwise (which covers notably the situation in which one register includes several smaller registers).

The RAM 97 comprises notably:

- in a register 970, the operating program of the microprocessor 91;
- in a register 971, information representative of the reference VA measurements 21 and of the reference blur data 22;
- in a register 972, information representative of the generic model 23;
- in a register 973, information representative of the specific model 24;
- in a register 974, information relevant to the refractive error, including information representative of the Rx estimation 25A and/or parameter values 25B.

When switched-on, the microprocessor 91 loads and executes the instructions of the program contained in the RAM 97.

**Claims**

1.  A device (1; 1A; 1B; 1C) for estimating a refractive error (Rx) of an eye (60) of a user from visual acuity of said user's eye, **characterized in that** said device includes:

    - at least one input (11) adapted to receive at least two measurements (21) of the visual acuity of said user's eye, called reference visual acuity measurements, and data (22) related to at least two distinct levels of blur respectively associated with the reference visual acuity measurements, called reference blur data, the reference visual acuity measurements being performed within a reference measurement period (T0) by imposing said respective levels of blur on said user's eye,
    - at least one processor configured for particularizing into a specific model (24) for said user's eye a generic model (23) involving a theoretical visual acuity (*VA)*, theoretical blur data (*Dist; θ, D*) and a theoretical refractive error (*S, C, α*), from the reference visual acuity measurements and the reference blur data, and for producing information (25) relevant to the refractive error of said user's eye from said specific model,
    - at least one output (15) adapted to provide said information relevant to the refractive error of said user's eye.

2.  The device (1; 1A; 1B; 1C) for estimating the refractive error of said user's eye according to claim 1, **characterized in that** the produced information relevant to the refractive error includes an estimation (25A) of the refractive error of said user's eye during the reference measurement period (T0).

3.  The device (1; 1B; 1C) for estimating the refractive error of said user's eye according to claim 1 or claim 2, **characterized in that** the produced information relevant to the refractive error includes at least one parameter value (25B) relevant to the specific model, said at least one parameter value enabling an estimation (28) of the refractive error of said user's eye after the reference measurement period, called later refractive error estimation, to be produced from at least one measurement (26) of the visual acuity of said user's eye after the reference measurement period (T0), called later visual acuity measurement.

4. The device (1; 1C) for estimating the refractive error of said user's eye according to claim 3, **characterized in that** said at least one input (11) is adapted to receive said at least one later visual acuity measurement (26), said at least one processor is configured for producing the later refractive error estimation (28) from the specific model (24) and from said at least one later visual acuity measurement, and said at least one output (15) is adapted to provide the later refractive error estimation (28).

5. The device (1; 1B; 1C) for estimating the refractive error of said user's eye according to claim 3 or claim 4, **characterized in that** the reference blur data (22) include at least one measurement of the refractive error of said user's eye during the reference measurement period.

6. The device (1; 1A; 1B; 1C) for estimating the refractive error of said user's eye according to any of the preceding claims, **characterized in that** said levels of blur are imposed at least partly by performing the reference visual acuity measurements (21) at respectively at least two reference distances (*Dist1, Dist2, Dist3*) from said user's eye to a test target, and the theoretical blur data include a theoretical distance (*Dist*) from said user's eye.

7. The device (1; 1A; 1B; 1C) for estimating the refractive error of said user's eye according to claim 6, **characterized in that** the generic model (23) involves successively, starting and moving away from said user's eye, three areas relevant to the theoretical visual acuity of said user's eye and consisting in a closest area (A1-3; A2-3; A3-3; A5-3), an accommodation area (A1-2; A2-2; A3-2; A5-2) and a farthest area (A1-1; A2-1; A5-1), the accommodation area being associated with a flat range of the theoretical visual acuity, and the closest area and farthest area being associated with variable values of the theoretical visual acuity, said levels of blur being imposed at least partly by performing the reference visual acuity measurements (21) at respectively said reference distances including at least one distance (*Dist2*) corresponding to the accommodation area and at least one distance (*Dist1, Dist3*) corresponding to at least one of the closest area and the farthest area.

8. The device (1; 1A; 1B; 1C) for estimating the refractive error of said user's eye according to any of the preceding claims, **characterized in that** at least one of said levels of blur is imposed at least partly by performing at least one of the reference visual acuity measurements (21) at respectively at least one reference direction angle (0°, 90°; $\theta$) of an oriented test target (55C), the theoretical blur data include a theoretical direction angle with respect to a predetermined axis, and said at least one processor is configured for particularizing the generic model (23) also from said at least one reference direction angle.

9. The device (1; 1A; 1B; 1C) for estimating the refractive error of said user's eye according to claim 8, **characterized in that** the refractive error of said user's eye having a spherical component, a cylindrical component and a cylinder axis, and the theoretical refractive error having a theoretical spherical component ($S$), a theoretical cylindrical component ($C$) and a theoretical cylinder axis ($\alpha$):

    - said at least one input (11) is adapted to receive at least one of the reference visual acuity measurements with respect to a non-oriented test target (55A), said at least one of the reference visual acuity measurements at respectively said at least one reference direction angle of the oriented test target (55C) and the respectively associated reference blur data (22), said reference blur data further including information on at least one offset (0°, 90°; $\theta$) of the cylinder axis with respect to the predetermined axis,
    - said at least one processor is configured for particularizing the generic model (23) into the specific model (24) from said reference visual acuity measurements (21) with respect to both the non-oriented test target and the oriented test target and from said reference blur data (22), and for producing information relevant to the spherical component and the cylindrical component of the refractive error of said user's eye,
    - said at least one output (15) is adapted to provide said information relevant to said spherical component and said cylindrical component.

10. The device (1; 1B; 1C) for estimating the refractive error of said user's eye according to any of the preceding claims, **characterized in that** at least one of said levels of blur is imposed at least partly by performing at least one of the reference visual acuity measurements with respectively at least one lens arranged in front of said user's eye so as to cause at least one predetermined optical power correction (+0.25 D, +0.5 D; [-4 D, -0.25 D]), and the theoretical blur data include a theoretical optical power correction (*DiffS1, DiffS3*) imposed on said user's eye.

11. The device (1; 1B; 1C) for estimating the refractive error of said user's eye according to claim 10, **characterized in**

**that** in the generic model (23), a variation of the theoretical refractive error (ΔS) is equated to the theoretical optical power correction (*DiffS1*).

12. The device (1; 1A; 1B; 1C) for estimating the refractive error of said user's eye according to any of the preceding claims, **characterized in that**:

- said at least one input (11) is adapted to receive dimensional data on a pupil of said user's eye (60) during said reference visual acuity measurements, called reference pupil data,
- the generic model (23) involving a theoretical pupil dimension (*D*), said at least one processor is configured for particularizing the generic model into the specific model (24) also in function of the reference pupil data.

13. An automatic module for myopia control including a device (1; 1A; 1B; 1C) for estimating a refractive error (Rx) of an eye (60) of a user from visual acuity of said user's eye, **characterized in that** said device includes:

- at least one input (11) adapted to receive at least two measurements (21) of the visual acuity of said user's eye, called reference visual acuity measurements, and data (22) related to at least two distinct levels of blur respectively associated with the reference visual acuity measurements, called reference blur data, the reference visual acuity measurements being performed within a reference measurement period (T0) by imposing said respective levels of blur on said user's eye,
- at least one processor configured for particularizing into a specific model (24) for said user's eye a generic model (23) involving a theoretical visual acuity (*VA*), theoretical blur data (*Dist; θ; D*) and a theoretical refractive error (*S, C, α*), from the reference visual acuity measurements and the reference blur data, and for producing information (25) relevant to the refractive error of said user's eye from said specific model,
- at least one output (15) adapted to provide said information relevant to the refractive error of said user's eye.

14. A method for estimating by at least one processor a refractive error (Rx) of an eye (60) of a user from visual acuity of said user's eye, **characterized in that** said method includes:

- receiving (81) at least two measurements (21) of the visual acuity of said user's eye, called reference visual acuity measurements, and data (22) related to at least two distinct levels of blur respectively associated with the reference visual acuity measurements, called reference blur data, the reference visual acuity measurements being performed within a reference measurement period (T0) by imposing said respective levels of blur on said user's eye,
- particularizing (82) into a specific model (24) for said user's eye a generic model (23) involving a theoretical visual acuity (*VA*), theoretical blur data (*Dist; θ; D*) and a theoretical refractive error (*S, C, α*), from the reference visual acuity measurements and the reference blur data, and producing information (25) relevant to the refractive error of said user's eye from said specific model,
- providing (85) said information relevant to the refractive error of said user's eye.

15. A computer program comprising software code adapted to perform a method for estimating a refractive error of an eye (60) of a user from visual acuity of said user's eye when said software code is executed by a processor, said method including:

- receiving (81) at least two measurements (21) of the visual acuity of said user's eye, called reference visual acuity measurements, and data (22) related to at least two distinct levels of blur respectively associated with the reference visual acuity measurements, called reference blur data, the reference visual acuity measurements being performed within a reference measurement period (T0) by imposing said respective levels of blur on said user's eye,
- particularizing (82) into a specific model (24) for said user's eye a generic model (23) involving a theoretical visual acuity (*VA*), theoretical blur data (*Dist; θ; D*) and a theoretical refractive error (*S, C, α*), from the reference visual acuity measurements and the reference blur data, and producing information (25) relevant to the refractive error of said user's eye from said specific model,
- providing (85) said information relevant to the refractive error of said user's eye.

**Fig 1**

Fig 2

**Fig 3**

**Fig 4**

**Fig 5**

**Fig 6**

Fig 7

**Fig 8**

**Fig 9**

55A

**Fig 10A**

55B

**Fig 10B**

55C

**Fig 10C**

55D

**Fig 10D**

55E

**Fig 10E**

55F

**Fig 10F**

**Fig 11**

**Fig 12A**

**Fig 12B**

**Fig 13**

**Fig 14**

Europäisches
Patentamt

European
Patent Office

Office européen
des brevets

# EUROPEAN SEARCH REPORT

Application Number

EP 23 30 5416

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/120402 A1 (LIMON OFER [IL]) 5 May 2016 (2016-05-05) | 1-5, 13-15 | INV. A61B3/028 |
| Y | * paragraphs [0038] - [0040]; claims 1-6, 15-17 * | 6,7, 10-12 | |
| A | | 8,9 | |
| | ----- | | |
| Y | US 2021/330181 A1 (POLAT URI [IL] ET AL) 28 October 2021 (2021-10-28) * paragraphs [0124], [0125], [0244], [0245]; figures 1,2 * | 6,7,12 | |
| | ----- | | |
| Y | US 10 548 473 B2 (ESSILOR INT [FR]) 4 February 2020 (2020-02-04) * column 5, line 40 ff.; figures 2,3 * | 6,7,10, 11 | |
| | ----- | | |
| A | JP 2022 117780 A (KYUSHU INST TECH) 12 August 2022 (2022-08-12) * paragraph [0008] * | 1-15 | |
| | ----- | | |

**TECHNICAL FIELDS SEARCHED (IPC)**

A61B

The present search report has been drawn up for all claims

| Place of search | Date of completion of the search | Examiner |
|---|---|---|
| Munich | 20 September 2023 | Fischer, Martin |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or after the filing date
D : document cited in the application
L : document cited for other reasons
.................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 30 5416

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2023

| Patent document cited in search report | Publication date | Patent family member(s) | Publication date |
|---|---|---|---|
| US 2016120402 A1 | 05-05-2016 | AU 2014276340 A1 | 24-12-2015 |
| | | BR 112015030406 A2 | 25-07-2017 |
| | | CA 2914456 A1 | 11-12-2014 |
| | | CN 105764405 A | 13-07-2016 |
| | | CN 110251066 A | 20-09-2019 |
| | | DK 3003121 T3 | 16-08-2021 |
| | | EP 3003121 A1 | 13-04-2016 |
| | | ES 2886136 T3 | 16-12-2021 |
| | | JP 6453859 B2 | 16-01-2019 |
| | | JP 6612959 B2 | 27-11-2019 |
| | | JP 2016523132 A | 08-08-2016 |
| | | JP 2019069180 A | 09-05-2019 |
| | | KR 20160017079 A | 15-02-2016 |
| | | PT 3003121 T | 06-09-2021 |
| | | RU 2706372 C1 | 18-11-2019 |
| | | RU 2015152290 A | 14-07-2017 |
| | | US 2016120402 A1 | 05-05-2016 |
| | | US 2017079523 A1 | 23-03-2017 |
| | | US 2019307324 A1 | 10-10-2019 |
| | | WO 2014195951 A1 | 11-12-2014 |
| US 2021330181 A1 | 28-10-2021 | US 2021330181 A1 | 28-10-2021 |
| | | WO 2020039426 A1 | 27-02-2020 |
| US 10548473 B2 | 04-02-2020 | BR 112017023251 A2 | 21-08-2018 |
| | | CN 108307619 A | 20-07-2018 |
| | | EP 3313263 A1 | 02-05-2018 |
| | | US 2018116500 A1 | 03-05-2018 |
| | | WO 2016207684 A1 | 29-12-2016 |
| JP 2022117780 A | 12-08-2022 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2020176784 A
- WO 2019089600 A
- WO 2014164020 A, S. P. Lee and A. Dallek
- WO 2014195951 A, O. Limon, O. K. Ancri and A. Zlotnik
- WO 2022013410 A

### Non-patent literature cited in the description

- **J. A. GÓMEZ-PEDRERO ; J. ALONSO.** Phenomenological model of visual acuity. *J. Biomed. Opt.,* 2016, vol. 21 (12), 125005
- Influence of combined power error and astigmatism on visual acuity. **C. FAUQUIER et al.** Vision Science and its Applications, Technical Digest Series. Optica Publishing Group, 1995
- **V. V. MOLEBNY et al.** Eye aberrations analysis with Zernike polynomials. *Proceedings of SPIE,* June 1998, 3246
- **M. BACH.** The Freiburg Visual Acuity Test - Automatic Measurement of Visual Acuity. *Optom. Vis. Sci.,* 1996, vol. 73, 49-53
- **C. PIECH et al.** The Stanford Acuity Test: A Precise Vision Test Using Bayesian Techniques and a Discovery in Human Visual Response. *Proceedings of the AAAI Conference on Artificial Intelligence,* 2020, vol. 34 (01), 471-479